# EUROPEAN PATENT APPLICATION

(11) **EP 2 527 501 A1**
(43) Date of publication of application: **28.11.2012**
(21) Application number: 12180869.5
(22) Date of filing: 15.11.2007
(51) Int. Cl.: C40B 40/06, A61K 31/65, A61K 31/704, A61P 35/00, C07H 21/00, C12Q 1/68, C40B 30/00, C12P 19/34, C40B 40/14

(54) **Polymorphisms predictive of anthracycline-induced cardiotoxicity**

(30) Priority: 15.11.2006 US 858924 P
(62) Divisional of application: 11150040.1
(71) Applicant: THE UNIVERSITY OF BRITISH COLUMBIA, Vancouver, British Columbia V6T 1Z3 (CA)
(72) Inventor: Carleton, Bruce, North Vancouver British Columbia V7R 2G2 (CA); Hayden, Michael, Vancouver British Columbia V6R 1W9 (CA); Ross, Colin, Burnaby British Columbia V5B 2H7 (CA)
(74) Representative: Henriksson, Dan Ragnar Mikael

(57) **Abstract**

Provided are methods, nucleic acids, and arrays for assessing the susceptibility of a subject to the development of cardiotoxicity in response to receiving one or more anthracycline compounds, the method including determining the presence or absence of one or more polymorphisms, wherein the presence or absence of one or more such polymorphisms is indicative of susceptibility to the development of cardiotoxicity.

## Description

### FIELD OF THE INVENTION

This invention relates to the field of genetic markers for adverse drug reactions. More specifically, methods and compositions useful for identifying individuals that may be at risk for an adverse drug reaction.

### BACKGROUND

Adverse drug reactions (ADRs) are a significant cause of illness, hospitalization and death for both children and adults in the Western world (LAZAROU et al JAMA 1998; PIRMOHAMED et al, BMJ 2004). Estimates suggest that 15% of hospitalized children experience an ADR. Those that do survive the ADR may be left disabled (MITCHELL et al., 1988 Pediatrics 82:24-9; MARTINEZ-MIR et al., 1999. Br J Clin Pharmacol 47 :681-8).

Many approved drugs used in children are untested in pediatric populations. While it is known that children metabolize drugs differently than adults, in many cases pediatric dosage forms are not available. This is of particular concern with chemotherapy drugs, which may frequently be supplied as a single-dose package, and in combination with other agents, excipients and the like. Pediatric populations also represent a more varied population, and this increased variability may be due to developmental differences in the normal expression of drug metabolism genes.

Genetic factors are involved in variability in drug response - ranging from 20-95% in some studies. Age, sex, body weight, health, medical history and the like may be accounted for, but patient genotype is largely an unknown factor (EVANS et al 2003. NEJM 348:538-549; WEINSHILBOUM 2003. NEJM 348:529-537).

Anthracyclines are used as cytotoxic agents in chemotherapeutic protocols in both children and adults, for a variety of neoplasms. Examples of anthracyclines and anthracycline analogues include daunorubicin, doxorubicin, idarubicin and epirubicin. For example, anthracyclines may be used in the treatment of solid and hematologic cancers, such as breast cancer, acute myeloid leukemia, acute lymphoblastic leukemia, multiple myeloma, Hodgkin's disease or non-Hodgkin's lymphoma.

Cardiotoxicity is a serious problem in patient populations receiving anthracyclines, particularly pediatric patients (LIPSHULTZ 2006. Seminars in Oncology 33:S8-S14). Anthracycline-induced cardiotoxicity may result in cardiomyopathy and congestive heart failure and may be irreversible. Anthracycline-induced cardiotoxicity may be characterized by reduced ventricular wall thickness and mass, indicative of decreased cardiac muscle and depressed ventricular contractility. Increased and cumulative dose, nature of the particular anthracycline, administration route, age, sex and prior radiation treatment may affect onset and severity of cardiotoxicity. Administration of enalapril, dexrazoxane or antioxidants such as vitamin E, coenzyme Q10, carnitine, or glutathione, for example may be beneficial in preventing or reducing cardiac injury during chemotherapy. Other agents that may be administered to reduce anthracycline cardiotoxicity are described (WOUTERS et al 2005. Br. J Hematol 131:561-578).

Dose limits have been empirically set in the clinic, above which the cardiotoxicity is deemed to be unacceptable. Subclinical and clinical cardiotoxicity may occur below these doses (JOHNSON 2006. Seminars in Oncology 33:S33-70) and affect current and subsequent therapeutic regimens. Liposomal anthracycline compositions may demonstrate reduced cardiotoxicity (EWER et al 2004. Seminars in Oncology 31:161-181).

Proteomic methods have been developed for early detection of drug-induced cardiotoxicity (PETRICOIN et al 2004. Toxicol Pathol 32:122-30).

Some polymorphisms in NAD(P)H oxidase are associated with anthracycline-induced cardiotoxicity (WOJNOWSKI et al 2005. Circulation 112:3754-3762).

Genotype has been shown to alter response to therapeutic interventions. Genentech's HERCEPTIN® was not effective in its overall Phase III trial but was shown to be effective in a genetic subset of subjects with human epidermal growth factor receptor 2 (HER2)-positive metastatic breast cancer. Similarly, Novartis' GLEEVEC® is only indicated for the subset of chronic myeloid leukemia subjects who carry a reciprocal translocation between chromosomes 9 and 22.

### SUMMARY

This invention is based in part on the identification that the particular nucleotide (allele) or genotype at the site of a given SNP may be associated with an increased likelihood of cardiotoxicity ('risk genotype') or an decreased likelihood of cardiotoxicity ('decreased risk genotype').

This invention is also based in part on the surprising discovery that rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441 SNPs alone or in combination are useful in predicting a subject's risk of cardiotoxicity following anthracycline treatment. Whereby the subjects having a decreased risk genotype are less likely to experience cardiotoxicity and subjects having a risk genotype are more likely to experience cardiotoxicity from the same treatment. Furthermore, there are provided herein rs138054; rs2071885; rs1229863; rs10509681; rs6499244; rs17863783; rs4148919; rs7785246; rs35607; rs16968478; rs11000122; rs4736349; rs741999; rs1677693; rs1845556; rs 1149222; rs1910465; rs7441743; rs10786172; and rs2107441 SNPs and SNPs in linkage disequilibrium (LD) thereto, which are also useful in predicting the response of a subject will have to anthracycline treatment.

In accordance with one aspect of the invention, methods are provided for screening a subject having a neoplastic disease for cardiotoxicity risk, the method including: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for the subject, where the subject may be a candidate for anthracycline administration.

In accordance with a further aspect of the invention, methods are provided for determining cardiotoxicity risk from anthracycline administration, the method comprising: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for a subject receiving or about to receive one or more anthracyclines.

In accordance with a further aspect of the invention, methods are provided for treating a neoplastic disease in a subject in need thereof, the method including administering to the subject an anthracycline, wherein said subject has a reduced risk of developing cardiotoxicity, wherein cardiotoxicity is based on the identity of a single nucleotide polymorphism (SNP) at one or more of the following polymorphic sites: rs138054; rs2071885; rs1229863; rs 10509681; rs6499244; rs17863783; rs4148919; rs7785246; rs35607; rs16968478; rs11000122; rs4736349; rs741999; rs1677693; rs 1845556; rs1149222; rs1910465; rs7441743; rs 10786172; and rs2107441; or a polymorphic site in linkage disequilibrium thereto.

In accordance with a further aspect of the invention, methods are provided for treating a neoplastic disease in a subject in need thereof, the method comprising: selecting a subject having a reduced risk of developing cardiotoxicity, wherein cardiotoxicity is based on the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto; and administering to said subject an anthracycline.

In accordance with a further aspect of the invention, methods are provided for selecting a chemotherapeutic regimen for a subject, the chemotherapeutic regimen including one or more anthracyclines, the method including: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for the subject to assess the risk of cardiotoxicity.

In accordance with a further aspect of the invention, uses of an anthracycline in the manufacture of a medicament for the treatment of neoplastic disease, wherein the subjects treated may have a reduced cardiotoxicity risk genotype at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs 1910465; rs7441743; rs10786172; and rs2107441; or a polymorphic site in linkage disequilibrium thereto.

In accordance with a further aspect of the invention, methods are provided for use of an anthracycline in the manufacture of a medicament for the treatment of neoplastic disease in a subset of subjects, wherein the subset of subjects have a reduced cardiotoxicity risk genotype at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs 1149222; rs1910465; rs7441743; rs10786172; and rs2107441; or a polymorphic site in linkage disequilibrium thereto.

In accordance with a further aspect of the invention, methods are provided for selecting test subjects to determine the efficacy of a therapeutic regimen known to be useful or suspected of being useful, for the treatment of a neoplastic condition, the method including: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs 1910465; rs7441743; rs10786172; and rs2107441; or a polymorphic site in linkage disequilibrium thereto, for a test subject, where the test subject is a candidate for anthracycline administration; and separating test subjects based on their risk of cardiotoxicity.

In accordance with a further aspect of the invention, methods are provided for determining risk of cardiotoxicity for a therapeutic regimen known to be useful or suspected of being useful, for the treatment of a neoplastic condition, the method including: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for a test subject, where the test subject is a candidate for anthracycline administration; and separating test subjects based on their risk of cardiotoxicity prior to anthracycline administration.

In accordance with a further aspect of the invention, methods are provided for selecting a group of subjects for determining the side effects of a candidate drug known or suspected of being useful for the treatment of a neoplastic condition, the method comprising: determining a subject's genotype for a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs 1149222; rs1910465; rs7441743; rs10786172; and rs2107441; or a polymorphic site in linkage disequilibrium thereto, for each subject, wherein a subject's genotype is indicative of the subject's risk of cardiotoxicity following chemotherapeutic regimen administration; and sorting subjects based on genotype. The method may further include, administering the candidate drug to the subjects or a subset of subjects and assessing the degree of hearing loss in each subject. The method may also further include comparing the degree of hearing loss in response to the candidate drug based on genotype of the subject.

The anthracycline may be selected from one or more of the following: anthracycline antibiotics such as daunorubicin (daunomycin, rubidomycin), doxorubicin, idarubicin, epirubicin, mitoxantrone, carminomycin, esorubicin, quelamycin, aclarubicin, esorubicin, zorubicin, pirarubicin, amrubicin, iododoxorubicin, mitoxantrone and valrubicin or other anthracycline compounds described herein. The method may further include obtaining a biological sample or samples from a subject or subjects. The method may further include administering the anthracycline in accordance with the subject's risk of developing cardiotoxicity.

The identity of a single nucleotide polymorphism may be determined by one or more of the following techniques: restriction fragment length analysis; sequencing; micro-sequencing assay; hybridization; invader assay; gene chip hybridization assays; oligonucleotide ligation assay; ligation rolling circle amplification; 5' nuclease assay; polymerase proofreading methods; allele specific PCR; matrix assisted laser desorption ionization time of flight (MALDI-TOF) mass spectroscopy; ligase chain reaction assay; enzyme-amplified electronic transduction; single base pair extension assay; and reading sequence data.

In accordance with a further aspect of the invention, there are provided two or more oligonucleotides or peptide nucleic acids of about 10 to about 400 nucleotides that hybridize specifically to a sequence contained in a human target sequence consisting of a subject's cardiotoxicity associated gene sequence, a complementary sequence of the target sequence or RNA equivalent of the target sequence and wherein the oligonucleotides or peptide nucleic acids are operable in determining the presence or absence of two or more polymorphism(s) in the cardiotoxicity associated gene sequence selected from of the following polymorphic sites: rs 138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs 1910465; rs7441743; rs10786172; and rs2107441; or a polymorphic site in linkage disequilibrium thereto.

In accordance with a further aspect of the invention, there are provided two or more oligonucleotides or peptide nucleic acids selected from the group including of: (a) an oligonucleotide or peptide nucleic acid that hybridizes under high stringency conditions to a nucleic acid molecule comprising SEQ ID NO: having an A at position 121 but not to a nucleic acid molecule comprising SEQ ID NO: having a G at position 121; (b) an oligonucleotide or peptide nucleic acid that hybridizes under high stringency conditions to a nucleic acid molecule comprising SEQ ID NO: 1 having a G at position 121 but not to a nucleic acid molecule comprising SEQ ID NO: 1 having an A at position 121; (c) an oligonucleotide or peptide nucleic acid that hybridizes under high stringency conditions to a nucleic acid molecule comprising SEQ ID NO:2 having a G at position 121 but not to a nucleic acid molecule comprising SEQ ID NO:2 having a C at position 121; (d) an oligonucleotide or peptide nucleic acid that hybridizes under high stringency conditions to a nucleic acid molecule comprising SEQ ID NO:2 having a C at position 121 but not to a nucleic acid molecule comprising SEQ ID NO:2 having a G at position 121; (e) an oligonucleotide or peptide nucleic acid that hybridizes under high stringency conditions to a nucleic acid molecule comprising SEQ ID NO:3 having an A at position 121 but not to a nucleic acid molecule comprising SEQ ID NO:3 having a T at position 121; (f) an oligonucleotide or peptide nucleic acid that hybridizes under high stringency conditions to a nucleic acid molecule comprising SEQ ID NO:3 having a T at position 121 but not to a nucleic acid molecule comprising SEQ ID NO:3 having an A at position 121; (g) an oligonucleotide or peptide nucleic acid that hybridizes under high stringency conditions to a nucleic acid molecule comprising SEQ ID NO:4 having a G at position 121 but not to a nucleic acid molecule comprising SEQ ID NO:4 having an A at position 121; (h) an oligonucleotide or peptide nucleic acid that hybridizes under high stringency conditions to a nucleic acid molecule comprising SEQ ID NO:4 having an A at position 121 but not to a nucleic acid molecule comprising SEQ ID NO:4 having a G at position 121; (i) an oligonucleotide or peptide nucleic acid that hybridizes under high stringency conditions to a nucleic acid molecule comprising SEQ ID NO:5 having an A at position 121 but not to a nucleic acid molecule comprising SEQ ID NO:5 having a T at position 121; (j) an oligonucleotide or peptide nucleic acid that hybridizes under high stringency conditions to a nucleic acid molecule comprising SEQ ID NO:5 having a T at position 121 but not to a nucleic acid molecule comprising SEQ ID NO:5 having an A at position 121; (k) an oligonucleotide or peptide nucleic acid that hybridizes under high stringency conditions to a nucleic acid molecule comprising SEQ ID NO:6 having an A at position 101 but not to a nucleic acid molecule comprising SEQ ID NO:6 having a C at position 101; (1) an oligonucleotide or peptide nucleic acid that hybridizes under high stringency conditions to a nucleic acid molecule comprising SEQ ID NO:6 having a C at position 101 but not to a nucleic acid molecule comprising SEQ ID NO:6 having an A at position 101; (m) an oligonucleotide or peptide nucleic acid that hybridizes under high stringency conditions to a nucleic acid molecule comprising SEQ ID NO:7 having an A at position 101 but not to a nucleic acid molecule comprising SEQ ID NO:7 having a G at position 101; (n) an oligonucleotide or peptide nucleic acid that hybridizes under high stringency conditions to a nucleic acid molecule comprising SEQ ID NO:7 having a G at position 101 but not to a nucleic acid molecule comprising SEQ ID NO:7 having an A at position 101; (o) an oligonucleotide or peptide nucleic acid that hybridizes under high stringency conditions to a nucleic acid molecule comprising SEQ ID NO:8 having a G at position 121 but not to a nucleic acid molecule comprising SEQ ID NO:8 having a C at position 121; (p) an oligonucleotide or peptide nucleic acid that hybridizes under high stringency conditions to a nucleic acid molecule comprising SEQ ID NO:8 having a C at position 121 but not to a nucleic acid molecule comprising SEQ ID NO:8 having a G at position 121; (q) an oligonucleotide or peptide nucleic acid that hybridizes under high stringency conditions to a nucleic acid molecule comprising SEQ ID NO:9 having an A at position 121 but not to a nucleic acid molecule comprising SEQ ID NO:9 having a G at position 121; (r) an oligonucleotide or peptide nucleic acid that hybridizes under high stringency conditions to a nucleic acid molecule comprising SEQ ID NO:9 having a G at position 121 but not to a nucleic acid molecule comprising SEQ ID NO:9 having an A at position 121; (s) an oligonucleotide or peptide nucleic acid that hybridizes under high stringency conditions to a nucleic acid molecule comprising SEQ ID NO:10 having an A at position 101 but not to a nucleic acid molecule comprising SEQ ID NO:10 having a G at position 101; (t) an oligonucleotide or peptide nucleic acid that hybridizes under high stringency conditions to a nucleic acid molecule comprising SEQ ID NO:10 having a G at position 101 but not to a nucleic acid molecule comprising SEQ ID NO:10 having an A at position 101; (u) an oligonucleotide or peptide nucleic acid that hybridizes under high stringency conditions to a nucleic acid molecule comprising SEQ ID NO:11 having an A at position 121 but not to a nucleic acid molecule comprising SEQ ID NO:11 having a G at position 121; (v) an oligonucleotide or peptide nucleic acid that hybridizes under high stringency conditions to a nucleic acid molecule comprising SEQ ID NO: 11 having a G at position 121 but not to a nucleic acid molecule comprising SEQ ID NO:11 having an A at position 121; (w) an oligonucleotide or peptide nucleic acid that hybridizes under high stringency conditions to a nucleic acid molecule comprising SEQ ID NO:12 having an A at position 121 but not to a nucleic acid molecule comprising SEQ ID NO:12 having a G at position 121; (x) an oligonucleotide or peptide nucleic acid that hybridizes under high stringency conditions to a nucleic acid molecule comprising SEQ ID NO:12 having a G at position 121 but not to a nucleic acid molecule comprising SEQ ID NO:12 having an A at position 121; (y) an oligonucleotide or peptide nucleic acid that hybridizes under high stringency conditions to a nucleic acid molecule comprising SEQ ID NO:13 having an A at position 121 but not to a nucleic acid molecule comprising SEQ ID NO:13 having a G at position 121; (z) an oligonucleotide or peptide nucleic acid that hybridizes under high stringency conditions to a nucleic acid molecule comprising SEQ ID NO:13 having a G at position 121 but not to a nucleic acid molecule comprising SEQ ID NO:13 having an A at position 121; (aa) an oligonucleotide or peptide nucleic acid that hybridizes under high stringency conditions to a nucleic acid molecule comprising SEQ ID NO: 14 having an A at position 121 but not to a nucleic acid molecule comprising SEQ ID NO:14 having a C at position 121; (bb) an oligonucleotide or peptide nucleic acid that hybridizes under high stringency conditions to a nucleic acid molecule comprising SEQ ID NO:14 having a C at position 121 but not to a nucleic acid molecule comprising SEQ ID NO:14 having an A at position 121; (cc) an oligonucleotide or peptide nucleic acid that hybridizes under high stringency conditions to a nucleic acid molecule comprising SEQ ID NO:15 having an A at position 121 but not to a nucleic acid molecule comprising SEQ ID NO:15 having a G at position 121; (dd) an oligonucleotide or peptide nucleic acid that hybridizes under high stringency conditions to a nucleic acid molecule comprising SEQ ID NO:15 having a G at position 121 but not to a nucleic acid molecule comprising SEQ ID NO:15 having an A at position 121; (ee) an oligonucleotide or peptide nucleic acid that hybridizes under high stringency conditions to a nucleic acid molecule comprising SEQ ID NO: 16 having an A at position 121 but not to a nucleic acid molecule comprising SEQ ID NO:16 having a C at position 121; (ff) an oligonucleotide or peptide nucleic acid that hybridizes under high stringency conditions to a nucleic acid molecule comprising SEQ ID NO:16 having a C at position 121 but not to a nucleic acid molecule comprising SEQ ID NO:16 having an A at position 121; (gg) an oligonucleotide or peptide nucleic acid that hybridizes under high stringency conditions to a nucleic acid molecule comprising SEQ ID NO:17 having a G at position 121 but not to a nucleic acid molecule comprising SEQ ID NO:17 having an A at position 121; (hh) an oligonucleotide or peptide nucleic acid that hybridizes under high stringency conditions to a nucleic acid molecule comprising SEQ ID NO: 17 having an A at position 121 but not to a nucleic acid molecule comprising SEQ ID NO:17 having a G at position 121; (ii) an oligonucleotide or peptide nucleic acid that hybridizes under high stringency conditions to a nucleic acid molecule comprising SEQ ID NO:18 having an A at position 101 but not to a nucleic acid molecule comprising SEQ ID NO:18 having a G at position 101; (jj) an oligonucleotide or peptide nucleic acid that hybridizes under high stringency conditions to a nucleic acid molecule comprising SEQ ID NO:18 having a G at position 101 but not to a nucleic acid molecule comprising SEQ ID NO:18 having an A at position 101; (kk) an oligonucleotide or peptide nucleic acid that hybridizes under high stringency conditions to a nucleic acid molecule comprising SEQ ID NO:19 having an A at position 101 but not to a nucleic acid molecule comprising SEQ ID NO:19 having a G at position 101; (11) an oligonucleotide or peptide nucleic acid that hybridizes under high stringency conditions to a nucleic acid molecule comprising SEQ ID NO:19 having a G at position 101 but not to a nucleic acid molecule comprising SEQ ID NO:19 having an A at position 101; (mm) an oligonucleotide or peptide nucleic acid that hybridizes under high stringency conditions to a nucleic acid molecule comprising SEQ ID NO:20 having an A at position 121 but not to a nucleic acid molecule comprising SEQ ID NO:20 having a G at position 121; and (nn) an oligonucleotide or peptide nucleic acid that hybridizes under high stringency conditions to a nucleic acid molecule comprising SEQ ID NO:20 having a G at position 121 but not to a nucleic acid molecule comprising SEQ ID NO:20 having an A at position 121.

In accordance with a further aspect of the invention, an array of oligonucleotides or peptide nucleic acids attached to a solid support is provided, the array comprising two or more of the oligonucleotides or peptide nucleic acids set out herein.

In accordance with a further aspect of the invention, composition comprising an addressable collection of two or more oligonucleotides or peptide nucleic acids are provided, wherein the two or more oligonucleotides or peptide nucleic acids consisting essentially of two or more nucleic acid molecules set out in SEQ ID NO:1-20 or compliments, fragments, variants, or analogs thereof.

The oligonucleotides or peptide nucleic acids may further include one or more of the following: a detectable label; a quencher; a mobility modifier; a contiguous non-target sequence situated 5' or 3' to the target sequence or 5' and 3' to the target sequence.

In accordance with a further aspect of the invention, a kit is provided for determining a genotype at one or more of the following polymorphic sites: rs 138054; rs2071885; rs 1229863; rs 10509681; rs6499244; rs17863783; rs4148919; rs7785246; rs35607; rs16968478; rs11000122; rs4736349; rs741999; rs1677693; rs1845556; rs1149222; rs 1910465; rs7441743; rs10786172; and rs2107441; or a polymorphic site in linkage disequilibrium thereto, in a subject to assess the subject's risk of cardiotoxicity following anthracycline administration, by distinguishing alternate nucleotides at the polymorphic site; or a labeled oligonucleotide having sufficient complementary to the polymorphic site so as to be capable of hybridizing distinctively to said alternate. The kit may further include an oligonucleotide or a set of oligonucleotides operable to amplify a region including the polymorphic site. The kit may further include a polymerization agent. The kit may further include instructions for using the kit to determine genotype.

In accordance with another aspect of the invention, there is provided a commercial package containing, as active pharmaceutical ingredient, use of anthracycline, or a pharmaceutically acceptable salt thereof, together with instructions for its use for the curative or prophylactic treatment of a neoplastic disease in a subject, wherein the subject treated has a reduced risk polymorphism in one or more of the following polymorphic sites: rs138054; rs2071885; rs1229863; rs10509681; rs6499244; rs17863783; rs4148919; rs7785246; rs35607; rs16968478; rs 11000122; rs4736349; rs741999; rs1677693; rs1845556; rs1149222; rs1910465; rs7441743; rs10786172; and rs2107441; or a polymorphic site in linkage disequilibrium thereto.

In accordance with another aspect of the invention, there is provided a commercial package containing, as active pharmaceutical ingredient, use of an anthracycline, or a pharmaceutically acceptable salt thereof, together with instructions for its use for the curative or prophylactic treatment of a neoplastic disease, wherein the subject treated may have a decreased risk polymorphism in in one or more of the following polymorphic sites: rs 138054; rs2071885; rs1229863; rs10509681; rs6499244; rs17863783; rs4148919; rs7785246; rs35607; rs16968478; rs 11000122; rs4736349; rs741999; rs1677693; rs 1845556; rs1149222; rs1910465; rs7441743; rs10786172; and rs2107441; or a polymorphic site in linkage disequilibrium thereto. Alternatively, the subject treated may have a risk polymorphism in in one or more of the above polymorphic sites, whereby cardiotoxicity is monitored and/or the treatment is adjusted accordingly.

The oligonucleotides or peptide nucleic acids may further include one or more of the following: a detectable label; a quencher; a mobility modifier; a contiguous non-target sequence situated 5' or 3' to the target sequence or 5' and 3' to the target sequence. The oligonucleotides or peptide nucleic acids may alternatively be of about 10 to about 400 nucleotides, about 15 to about 300 nucleotides. The oligonucleotides or peptide nucleic acids may alternatively be of about 20 to about 200 nucleotides, about 25 to about 100 nucleotides. The oligonucleotides or peptide nucleic acids may alternatively be of about 20 to about 80 nucleotides, about 25 to about 50 nucleotides. The genotype may be determined using a nucleic acid sample from the subject. Genotype may be determined using one or more of the following techniques: restriction fragment length analysis; sequencing; micro-sequencing assay; hybridization; invader assay; gene chip hybridization assays; oligonucleotide ligation assay; ligation rolling circle amplification; 5' nuclease assay; polymerase proofreading methods; allele specific PCR; matrix assisted laser desorption ionization time of flight (MALDI-TOF) mass spectroscopy; ligase chain reaction assay; enzyme-amplified electronic transduction; single base pair extension assay; and reading sequence data. A determination of whether a site is in linkage disequilibrium (LD) with another site may be determined based on an absolute r² value or D' value. When evaluating loci for LD those sites within a given population having a high degree of linkage disequilibrium (for example an absolute value for D' of ≥ 0.5 or r² > 0.5) are potentially useful in predicting the identity of an allele of interest (for example associated with the condition of interest). A high degree of linkage disequilibrium may be represented by an absolute value for D' of ≥ 0.6 or r² ≥ 0.6. Alternatively, a higher degree of linkage disequilibrium may be represented by an absolute value for D' of ≥0.7 or r² ≥ 0.7 or by an absolute value for D' of ≥0.8 or r² ≥ 0.8. Additionally, a high degree of linkage disequilibrium may be represented by an absolute value for D' of ≥ 0.85 or r ≥ 0.85 or by an absolute value for D' of ≥ 0.9 or r² ≥0.9. Two or more oligonucleotides or peptide nucleic acids may include 3 or more; 4 or more; 5 or more; 6 or more; 7 or more; 8 or more; 9 or more; 10 or more; 11 or more; 12 or more; 13 or more; 14 or more; 15 or more; 16 or more; 17 or more; 18 or more; 19 or more; or 20 or more.

Sequence variations may be assigned to a gene if mapped within 2 kb or more of an mRNA sequence feature. In particular, such a sequence may extend many kilobases (kb) from a gene and into neighbouring genes, where the LD within a region is strong.

### DETAILED DESCRIPTION

### 1. Definitions

In the description that follows, a number of terms are used extensively, the following definitions are provided to facilitate understanding of the various embodiments of the invention.

An "anthracycline compound" or "anthracycline" or "anthracycline derivatives" or "anthracycline analogues" as used herein is typically an anthraquinone core attached to a carbohydrate moiety and derivative thereof (see for example, FAN et al. J. Org. Chem. (2007) 72:2917-2928; Goodman and Gilman's The Pharmacological Basis of Therapeutics 8th edition editors Alfred Goodman Gilman, Theodore Rail, Alan Nies, Palmer Taylor. Pergamon Press. 1990 pg 1241-1244). For example, include anthracycline antibiotics such as daunorubicin (daunomycin, rubidomycin), doxorubicin, idarubicin, epirubicin, mitoxantrone, carminomycin, esorubicin, quelamycin, aclarubicin, esorubicin, zorubicin, pirarubicin, amrubicin, iododoxorubicin, mitoxantrone and valrubicin.

"Genetic material" includes any nucleic acid and can be a deoxyribonucleotide or ribonucleotide polymer in either single or double-stranded form.

A nucleotide represented by the symbol M may be either an A or C, a nucleotide represented by the symbol W may be either an T/U or A, a nucleotide represented by the symbol Y may be either an C or T/U, a nucleotide represented by the symbol S may be either an G or C, while a nucleotide represented by the symbol R may be either an G or A, and a nucleotide represented by the symbol K may be either an G or T/U. Similarly, a nucleotide represented by the symbol V may be either A or G or C, while a nucleotide represented by the symbol D may be either A or G or T, while a nucleotide represented by the symbol B may be either G or C or T, and a nucleotide represented by the symbol H may be either A or C or T.

A "polymorphic site" or "polymorphism site" or "polymorphism" or "single nucleotide polymorphism site" (SNP site) or single nucleotide polymorphism" (SNP) as used herein is the locus or position with in a given sequence at which divergence occurs. A "polymorphism" is the occurrence of two or more forms of a gene or position within a gene (allele), in a population, in such frequencies that the presence of the rarest of the forms cannot be explained by mutation alone. The implication is that polymorphic alleles confer some selective advantage on the host. Polymorphic sites have at least two alleles, each occurring at frequency of greater than 1%, and may be greater than 10% or 20% of a selected population. Polymorphic sites may be at known positions within a nucleic acid sequence or may be determined to exist. Polymorphisms may occur in both the coding regions and the noncoding regions (for example, promoters, introns or untranslated regions) of genes. Polymorphisms may occur at a single nucleotide site (SNPs) or may involve an insertion or deletion as described herein.

A "risk genotype" as used herein refers to an allelic variant (genotype) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs 1910465; rs7441743; rs10786172; and rs2107441; or a polymorphic site in linkage disequilibrium thereto, for the subject as described herein, as being indicative of a increased likelihood of cardiotoxicity following administration of an anthracycline. The risk genotype may be determined for either the haploid genotype or diploid genotype, provided that at least one copy of a risk allele is present. Risk genotype may be an indication of an increased risk of cardiotoxicity. Subjects having one copy (heterozygotes) or two copies (homozygotes) of the risk allele are considered to have the "risk genotype" even though the degree to which the subjects is at risk cardiotoxicity may increase, depending on whether the subject is a homozygote rather than a heterozygote. Such "risk alleles" or "risk genotypes" may be selected from the following: rs138054G; rs2071885C; rs1229863T; rs10509681C; rs6499244T; rs17863783T; rs4148919C; rs7785246G; rs35607T; rs16968478G; rs11000122T; rs4736349T; rs741999A; rs1677693A; rs1845556T; rs1149222G; rs1910465T; rs7441743A; rs10786172A; and rs2107441G; or a polymorphic site in linkage disequilibrium thereto (risk alleles on the forward strand).

A "decreased risk genotype" as used herein refers to an allelic variant (genotype) at one or more of the following polymorphic sites: rs 138054; rs2071885; rs 1229863; rs 10509681; rs6499244; rs17863783; rs4148919; rs7785246; rs35607; rs16968478; rs11000122; rs4736349; rs741999; rs 1677693; rs 1845556; rs 1149222; rs1910465; rs7441743; rs 10786172; and rs2107441 or a polymorphic site in linkage disequilibrium thereto, for the subject as described herein, as being indicative of a decreased likelihood of cardiotoxicity following administration of an anthracycline. "Decreased risk alleles" or "decreased risk genotypes" or "reduced risk genotypes" may be selected from the following: rs138054A; rs2071885G; rs1229863A; rs 10509681T; rs6499244A; rs17863783G; rs4148919T; rs7785246C; rs35607C; rs16968478A; rs11000122C; rs4736349C; rs741999G; rs1677693C; rs1845556C; rs1149222T; rs1910465C; rs7441743G; rs 10786172G; and rs2107441A; or a polymorphic site in linkage disequilibrium thereto (decreased risk alleles on the forward strand).

A "clade" is a group of haplotypes that are closely related phylogenetically. For example, if haplotypes are displayed on a phylogenetic (evolutionary) tree a clade includes all haplotypes contained within the same branch.

The pattern of a set of markers along a chromosome is referred to as a "Haplotype". Accordingly, groups of alleles on the same small chromosomal segment tend to be transmitted together. Haplotypes along a given segment of a chromosome are generally transmitted to progeny together unless there has been a recombination event. Absence of a recombination event, haplotypes can be treated as alleles at a single highly polymorphic locus for mapping.

As used herein "haplotype" is a set of alleles of closely linked loci on a chromosome that tend to be inherited together. Such allele sets occur in patterns, which are called haplotypes. Accordingly, a specific SNP or other polymorphism allele at one SNP site is often associated with a specific SNP or other polymorphism allele at a nearby second SNP site or other polymorphism site. When this occurs, the two SNPs or other polymorphisms are said to be in Linkage Disequilibrium (LD) because the two SNPs or other polymorphisms are not just randomly associated (i.e. in linkage equilibrium).

In general, the detection of nucleic acids in a sample depends on the technique of specific nucleic acid hybridization in which the oligonucleotide is annealed under conditions of "high stringency" to nucleic acids in the sample, and the successfully annealed oligonucleotides are subsequently detected (see for example Spiegelman, S., Scientific American, Vol. 210, p. 48 (1964)). Hybridization under high stringency conditions primarily depends on the method used for hybridization, the oligonucleotide length, base composition and position of mismatches (if any). High-stringency hybridization is relied upon for the success of numerous techniques routinely performed by molecular biologists, such as high-stringency PCR, DNA sequencing, single strand conformational polymorphism analysis, and in situ hybridization. In contrast to Northern and Southern hybridizations, these aforementioned techniques are usually performed with relatively short probes (e.g., usually about 16 nucleotides or longer for PCR or sequencing and about 40 nucleotides or longer for in situ hybridization). The high stringency conditions used in these techniques are well known to those skilled in the art of molecular biology, and examples of them can be found, for example, in Ausubel et al., Current Protocols in Molecular Biology, John Wiley & Sons, New York, N.Y., 1998.

"Oligonucleotides" as used herein are variable length nucleic acids, which may be useful as probes, primers and in the manufacture of microarrays (arrays) for the detection and/or amplification of specific nucleic acids. Such DNA or RNA strands may be synthesized by the sequential addition (5'-3' or 3'-5') of activated monomers to a growing chain, which may be linked to an insoluble support. Numerous methods are known in the art for synthesizing oligonucleotides for subsequent individual use or as a part of the insoluble support, for example in arrays (BERNFIELD MR. and ROTTMAN FM. J. Biol. Chem. (1967) 242(18):4134-43; SULSTON J. et al. PNAS (1968) 60(2):409-415; GILLAM S. et al. Nucleic Acid Res.(1975) 2(5):613-624; BONORA GM. et al. Nucleic Acid Res.(1990) 18(11):3155-9; LASHKARI DA. et al. Proc Nat Acad Sci (1995) 92(17):7912-5; MCGALL G. et al. PNAS (1996) 93(24):13555-60; ALBERT TJ. et al. Nucleic Acid Res.(2003) 31(7):e35; GAO X. et al. Biopolymers (2004) 73(5):579-96; and MOORCROFT MJ. et al. Nucleic Acid Res.(2005) 33(8):e75). In general, oligonucleotides are synthesized through the stepwise addition of activated and protected monomers under a variety of conditions depending on the method being used. Subsequently, specific protecting groups may be removed to allow for further elongation and subsequently and once synthesis is complete all the protecting groups may be removed and the oligonucleotides removed from their solid supports for purification of the complete chains if so desired.

"Peptide nucleic acids" (PNA) as used herein refer to modified nucleic acids in which the sugar phosphate skeleton of a nucleic acid has been converted to an N-(2-aminoethyl)-glycine skeleton. Although the sugar-phosphate skeletons of DNA/RNA are subjected to a negative charge under neutral conditions resulting in electrostatic repulsion between complementary chains, the backbone structure of PNA does not inherently have a charge. Therefore, there is no electrostatic repulsion. Consequently, PNA has a higher ability to form double strands as compared with conventional nucleic acids, and has a high ability to recognize base sequences. Furthermore, PNAs are generally more robust than nucleic acids. PNAs may also be used in arrays and in other hybridization or other reactions as described above and herein for oligonucleotides.

An "addressable collection" as used herein is a combination of nucleic acid molecules or peptide nucleic acids capable of being detected by, for example, the use of hybridization techniques or by any other means of detection known to those of ordinary skill in the art. A DNA microarray would be considered an example of an "addressable collection".

In general the term "linkage", as used in population genetics, refers to the co-inheritance of two or more nonallelic genes or sequences due to the close proximity of the loci on the same chromosome, whereby after meiosis they remain associated more often than the 50% expected for unlinked genes. However, during meiosis, a physical crossing between individual chromatids may result in recombination. "Recombination" generally occurs between large segments of DNA, whereby contiguous stretches of DNA and genes are likely to be moved together in the recombination event (crossover). Conversely, regions of the DNA that are far apart on a given chromosome are more likely to become separated during the process of crossing-over than regions of the DNA that are close together. Polymorphic molecular markers, like SNPs, are often useful in tracking meiotic recombination events as positional markers on chromosomes.

Furthermore, the preferential occurrence of a disease gene in association with specific alleles of linked markers, such as SNPs or other polymorphisms, is called "Linkage Disequilibrium" (LD). This sort of disequilibrium generally implies that most of the disease chromosomes carry the same mutation and the markers being tested are relatively close to the disease gene(s).

For example, in SNP-based association analysis and LD mapping, SNPs can be useful in association studies for identifying polymorphisms, associated with a pathological condition, such as sepsis. Unlike linkage studies, association studies may be conducted within the general population and are not limited to studies performed on related individuals in affected families. In a SNP association study the frequency of a given allele (i.e. SNP allele) is determined in numerous subjects having the condition of interest and in an appropriate control group. Significant associations between particular SNPs or SNP haplotypes and phenotypic characteristics may then be determined by numerous statistical methods known in the art.

Association analysis can either be direct or LD based. In direct association analysis, potentially causative SNPs may be tested as candidates for the pathogenic sequence. In LD based SNP association analysis, SNPs may be chosen at random over a large genomic region or even genome wide, to be tested for SNPs in LD with a pathogenic sequence or pathogenic SNP. Alternatively, candidate sequences associated with a condition of interest may be targeted for SNP identification and association analysis. Such candidate sequences usually are implicated in the pathogenesis of the condition of interest. In identifying SNPs associated with cardiotoxicity, candidate sequences may be selected from those already implicated in the pathway of the condition or disease of interest. Once identified, SNPs found in or associated with such sequences, may then be tested for statistical association with an individual's prognosis or susceptibility to the condition or to the side effect of a medication.

For an LD based association analysis, high density SNP maps are useful in positioning random SNPs relative to an unknown pathogenic locus. Furthermore, SNPs tend to occur with great frequency and are often spaced uniformly throughout the genome. Accordingly, SNPs as compared with other types of polymorphisms are more likely to be found in close proximity to a genetic locus of interest. SNPs are also mutationally more stable than variable number tandem repeats (VNTRs) and short tandem repeats (STRs).

In population genetics linkage disequilibrium refers to the "preferential association of a particular allele, for example, a mutant allele for a disease with a specific allele at a nearby locus more frequently than expected by chance" and implies that alleles at separate loci are inherited as a single unit (Gelehrter, T.D., Collins, F.S. (1990). Principles of Medical Genetics. Baltimore: Williams & Wilkens). Accordingly, the alleles at these loci and the haplotypes constructed from their various combinations serve as useful markers of phenotypic variation due to their ability to mark clinically relevant variability at a particular position (see Akey, J. et al. Eur J Hum Genet (2001) 9:291-300; and Zhang, K. et al. (2002). Am J Hum Genet. 71:1386-1394). This viewpoint is further substantiated by Khoury et al. ((1993). Fundamentals of Genetic Epidemiology. New York: Oxford University Press at p. 160) who state, "[w]henever the marker allele is closely linked to the true susceptibility allele and is in [linkage] disequilibrium with it, one can consider that the marker allele can serve as a proxy for the underlying susceptibility allele."

As used herein "linkage disequilibrium" (LD) is the occurrence in a population of certain combinations of linked alleles in greater proportion than expected from the allele frequencies at the loci. For example, the preferential occurrence of a disease gene in association with specific alleles of linked markers, such as SNPs, or between specific alleles of linked markers, are considered to be in LD. This sort of disequilibrium generally implies that most of the disease chromosomes carry the same mutation and that the markers being tested are relatively close to the disease gene(s). Accordingly, if the genotype of a first locus is in LD with a second locus (or third locus etc.), the determination of the allele at only one locus would necessarily provide the identity of the allele at the other locus. When evaluating loci for LD those sites within a given population having a high degree of linkage disequilibrium (i.e. an absolute value for r² ≥0.5) are potentially useful in predicting the identity of an allele of interest (i.e. associated with the condition of interest). A high degree of linkage disequilibrium may be represented by an absolute value for r² ≥ 0.6. Alternatively, a high degree of linkage disequilibrium may be represented by an absolute value for r² ≥ 0.7 or by an absolute value for r²≥ 0.8. Additionally, a high degree of linkage disequilibrium may be represented by an absolute value for r² ≥0.85 or by an absolute value for r² ≥ 0.9 or by an absolute value for r² ≥ 0.95. Accordingly, two SNPs that have a high degree of LD may be equally useful in determining the identity of the allele of interest or disease allele. Therefore, we may assume that knowing the identity of the allele at one SNP may be representative of the allele identity at another SNP in LD. Accordingly, the determination of the genotype of a single locus can provide the identity of the genotype of any locus in LD therewith and the higher the degree of linkage disequilibrium the more likely that two SNPs may be used interchangeably.

LD may be useful for genotype-phenotype association studies. For example, if a specific allele at one SNP site (e.g. "A") is the cause of a specific clinical outcome (e.g. call this clinical outcome "B") in a genetic association study then, by mathematical inference, any SNP (e.g. "C") which is in significant LD with the first SNP, will show some degree of association with the clinical outcome. That is, if A is associated (~) with B, i.e. A~B and C~A then it follows that C~B. Of course, the SNP that will be most closely associated with the specific clinical outcome, B, is the causal SNP - the genetic variation that is mechanistically responsible for the clinical outcome. Thus, the degree of association between any SNP, C, and clinical outcome will depend on LD between A and C.

Until the mechanism underlying the genetic contribution to a specific clinical outcome is fully understood, LD helps identify potential candidate causal SNPs and also helps identify a range of SNPs that may be clinically useful for prognosis of clinical outcome or of treatment effect. If one SNP within a gene is found to be associated with a specific clinical outcome, then other SNPs in LD will also have some degree of association and therefore some degree of prognostic usefulness.

Polymorphisms in linkage disequilibrium may be identified, for example, using the Haploview program (BARRETT JC. et al. Bioinformatics (2005) 21 (2):263-5 (http://www.broad.mit.edu/mpg/haploview/)) and the LD function in the Genetics Package in R (R Core Development Group, 2005 - R Development Core Team (www.R-project.org). Linkage Disequilibrium between markers may be defined using r² whereby all SNPs available on Hapmap.org (phase II) (cohort H), all SNPs genotyped internally using the Illumina Goldengate assay (cohort I) and SNPs may be sequenced using the Sequenom Iplex Platform (cohort S) for genes of interest. A minimum r² of 0.5 may be used as the cutoff to identify LD SNPs.

Numerous sites have been identified as polymorphic sites associated cardiotoxicity following anthracycline administration (see TABLE 1).

**TABLE 1. Single Nucleotide Polymorphisms Associated with Anthracycline-Induced Cardiotoxicitv**

| **Gene Symbol** | **SNP ID** | **SNP Position (BUILD 35)** | **Chromosome** | **SNP Alleles (* reverse strand)** | **Adverse Drug Reaction Predictive Variant** | **Odds Ratio** | **Chi Test P-value** |
|---|---|---|---|---|---|---|---|
| **SULT4A1** | rs138054 | 42544473 | 22 | [A/G] | G | 5.3 | 0.00015 |
| **SULT4A1** | rs2071885 | 42553744 | 22 | [G/C] | c | 5.0 | 0.00030 |
| **ADH1B/C** | rs1229863 | 100609564 | 4 | [T/A]* | A | 7.9 | 0.00034 |
| **CYP2C8** | rs10509681 | 96788739 | 10 | [A/G]* | G | 4.6 | 0.00039 |
| **NQ01_NF AT5** | rs6499244 | 68292772 | 16 | [A/T]* | A | 5.0 | 0.00083 |
| **UGT1A6/7/ 8/9/10** | rs17863783 | 234384277 | 2 | [A/C]* | A | 6.8 | 0.00088 |
| **CHST3** | rs4148919 | 73401619 | 10 | [A/G]* | G | 7.0 | 0.0026 |
| **TBXAS1** | rs7785246 | 138995358 | 7 | [G/C]* | C | 5.7 | 0.0027 |
| **ABCC1** | rs35607 | 16069981 | 16 | [A/G]* | A | 4.6 | 0.0037 |
| **CYP11A1** | rs16968478 | 72449864 | 15 | [A/G] | G | 8.0 | 0.0037 |
| **CHST3** | rs11000122 | 73392029 | 10 | [A/G]* | A | 3.9 | 0.0039 |
| **CYP11B1** | rs4736349 | 143964434 | 8 | [A/G]* | A | 3.3 | 0.0064 |
| **NCF4** | rs741999 | 35583773 | 22 | [A/G] | A | 3.3 | 0.0067 |
| **DHFR** | rs1677693 | 79972074 | 5 | [A*l*C] | A | 3.6 | 0.0079 |
| **UGT2B4** | rs 1845556 | 70535644 | 4 | [A/G]* | A | 3.2 | 0.0085 |
| **ABCB4** | rs1149222 | 86718426 | 7 | [A/C]* | C | 3.3 | 0.0091 |
| **SULT1E1** | rs1910465 | 70885302 | 4 | [A/G]* | A | 3.2 | 0.01 |
| **UGT2B4** | rs7441743 | 70540454 | 4 | [A/G] | A | 3.0 | 0.012 |
| **CYP2C19** | rs10786172 | 96571084 | 10 | [A/G] | A | 3.0 | 0.012 |
| **ABCC3** | rs2107441 | 46061819 | 17 | [A/G] | G | 3.0 | 0.013 |

**TABLE 2.** below shows the flanking sequences for the SNPs shown in TABLE 1 providing their rs designations and corresponding SEQ ID NO designations. Each polymorphism is at position 101 within the flanking sequence unless otherwise indicated, and identified in bold.

**TABLE 2. Sequence for Cardiotoxicity-Associated Polymorphisms Listed in TABLE 1, with SEQ ID NO designations**

| **Gene Symbol** | **SNP ID** | **SNP Alleles (* reverse strand)** | **ADR-Predictive Variant** | **GENOMIC SEQUENCE** | **SEQ ID NO:** |
|---|---|---|---|---|---|
| **SULT4A1** | rs138054 (at position 121) | [A/G] | G | TGTTCCCTATCCCCTTAGAGCACGCTTGGG CCTGAACTGATCTTGGGTTCTGTCGCCTGT CTACCCCACTGGAATGCAAACTCCACAGAG GCAGGGCTTTCTCTGTCCCCTGCCGCATCC RCAGTGCCGGGAACAGTGCCTGGCATGGAG CAGGTGCAGTCGAATGACCGACTCCATGAA GTGAGGTGAGTCACCCACCACGCAGGGAAG AGTGGACATGGGTAAACAGGCGCTGCCTGG G | 1 |
| **SULT4A1** | rs2071885 (at position 121) | [G/C] | C | CCCAGCACCCAGCTCTCCTGTCTGTCTCCC AGCAGACCTCCTCCCCATCCCAGCTCCCAC CCACTGCTACTCAACCTAAACCTGCTTGTC TGTCAGCCACTGACACTAGCAGCCCCACAG SCTGGCCTTGTCTGTCCCCTACATCGACAT GGTTTCCAGCTGGCACTCAGGCCTCTGCCC CACAGGAGCAACCTCACAGTCCACATCTGC ACCCCATCGTCTCTCCTCTTTCCCATGGGC C | 2 |
| **ADH1B/C** | rs1229863 (at position 121) | [T/A]* | A | CTGGATGGTCTTCATCTCTTGACCTTGTGA TCCTCCTGCCTCGGCCTCCCAAAGTGCTGG GATTACAGGCATGCACCTCCCATTCTGAAC GGGAGGTAGACTTTGCATAAAAAACTGCAT WTGAAAATCCTAGAGGGCCTTCAAACTCAT CAAGTCCATTTCCCAAACTTTACCATTGTG ATGGTTTGAATATTAGTCCTTCAAATCTCA ATTTGAAATTTACTTGCCATTGTAATAGTA T | 3 |
| **CYP2C8** | rs10509681 (at position 121) | [A/G]* | G | CTACGTGATGTCCACTACTTCTCCTCACTT CTGGACTTCTTTATAAATCAGATTATCTGT TTTGTTACTTCCAGGGCACAACCATAATGG CATTACTGACTTCCGTGCTACATGATGACA RAGAATTTCCTAATCCAAATATCTTTGACC CTGGCCACTTTCTAGATAAGAATGGCAACT TTAAGAAAAGTGACTACTTCATGCCTTTCT CAGCAGGTAATAGAAACTCGTTTCCATTTG T | 4 |
| **NQO1_N FAT5** | rs6499244 (at position 121) | [T/A]* | A | AAAGAAGGTTTAAATGGAAAAAGTAAAAGC TGGGACCTACTGAAACTATTTGTTTATGTG TTTTTGGAAATAGTGAAAAATGAAATTATA AATGATAAAAAGGCACTTTTCCATAGCATT WGATCTAGATATAAAATATTAATTTAAAAA ATTTCCTGAAAGAGAAAGTAAAAACATAAT TTTATTAATAAATGAGGCTAGAATGGCATA AAAATAAATGGGGATTAAAGAAGTAATTAG G | 5 |
| **UGT1A6/ 7/8/9/10** | rs17863783 | [C/A]* | A | TATCCACATCTCTCTTGAGGACAGCTGATG CGAGTTCTTCATACTTTGAAAACAGACAAT AAAATAGATAGGGCTCCAACAAATTAACAA GGAAGTTGGCMACTCGTTGGGAAAAAGTCA TGTGGTCTGAAAACTTTGTGTAGCACCTGG GAATGTAGGACACAGGGTCTGGGCTTCTGC TGAATGNATGCTCCAGGGAAC | 6 |
| **CHST3** | rs4148919 | [A/G]* | G | TCGGTAAAAGAAAGCAAGTGGGTGACCTG CGAGGTCACAGTCAGTTCTTCATCCTCTGG TGGGTGACACTGCTTCGTGACTGGCAGGTT CTAGATTTCTRTTCTCGGGGCCTCTTCAGA CACATCAAACCTGCAGGACAGGCCCTCAGC TTCTCTCCGTGTCTCTAACAAGCCCCTGGA AGTCTCCAGAGGAAAGCCAGG | 7 |
| **TBXAS1** | rs7785246 (at position 121) | [G/C]* | C | ATAAGGCTACCAGGGTTCCCATAGACCGCG TACTTCCCTTCAGTGGCAAGCGATAGTTGT TAGGTTTGTTGTTATTATGGCACTAGGGTT AGGGGAAGAGTTCATTCAATCATTTTATTT STGGGAGAGTCACTTCCTTCCCGGGAAGGC ATCGCTGAGCACCCACTCACGCAGCTCAGT AATGGACACACATCAATCTGATGTTTTGCT TTGTTTATACAACAAATAAGATTCTTCTTT C | 8 |
| **ABCC1** | rs35607 (at position 121) | [G/A)* | A | AAATGGTGGGAATACAGGCATGAGCCGCCG CGCCCGGCTGTAAGGTTGGGGTTTCTCAAA GCGTGGGTGGTCAAGACCTATCAGTGGGTT GTAAAATCAATCAAGTCAGACAAGAGCTAC RTTTTTTAAAAAAAGGATAGGAATGGCGGA GGAACAGGCTGAAGAGAACAGAGTATTTCG GAAAGATTCTTCCAGAGCATGGGAAAGCCC TGCTGCATTAAACTTGATTCATATACTCAA G | 9 |
| **CYP11A1** | rs16968478 | [A/G] | G | CCTGCCTCCCACATTCTGTAACTATCCTAT CATTCTCAGAGTAGAGGGTGTACACATTTA GACCCATCTAAACGTTGCCTAGGACGGGGA ACATGATTTTRTTCATTGCAGGCAGCTTGG ATGGATAAACAGAAAATCCAAATCACAGAC TTAAAGAAGATAGAAGTGTATTCTCTCTCC CATAAAGGATGTCCAAGGCTG | 10 |
| **CHST3** | rs11000122 (at position 121) | [G/A]* | A | GCTCCCAATGGCCTGAAAATGGCACTTTTG ATTTCAATATACTTAGAGACTTAGACGACT TTTGCCATCGCAGTGGAAATGGTCTGAGA TTCCTTATGTTCAGGCTTTCTTCACTCTCC RTAACCGCCCTTCCCTCTGCCAGTACTGCT CCACTTTCCAAATCCTACTCACCAGACTTG TCTCCCACCACCCCCGTCTCCCACCACCCC CGCAACAGCCCCAGCCGACAACTCCTCTTT C | 11 |
| **CYP11B1** | rs4736349 (at position 121) | [G/A]* | A | ACATGGTGCTGGCATCTTATCAGCTTCTGG TGAGGCCTGAGGAAGTTTACAATCATGGAG GAAGGCAAAGGGGGAGCCAGCGTACCAGAT GACGAGAGTGGGCATGAGAAAGCGAGGGGG RAAGTGCCACATTCTTTTGAACAACAGATC TCATGTGAACTCAGATAACTTATTCTCTAC TAAGGCGACACTCTCAGCCATTCATGAGGG ATCTGCCCCCATGATCCAGTACCTCCCACC A | 12 |
| **NCF4** | rs741999 (at position 121) | [G/A] | A | GCTCCGCTCCTTCCCTCCTCTGTAAAATGG GGACRGGAGAAGTACTTATGTCTCCGGGTG GTCATGCAGTCCCATCTGTAACAAACACAG CCCTAAAATCTCCATGAGGGCAAGGACTCT RCTCACCCTACATCCCAGTGCCCAGAACAG AGCCCGGCATACAGTAGGTGCTCAGTACTG CTCAATACACGGCCACTACCGTCGTCTGCA ATTATAGAGTTTTTTGATGGGCAGCATGAG A | 13 |
| **DHFR** | rs1677693 (at position 121) | [C/A] | A | TTTAAAATAATTATAGGAAAGAAATTCAGC AGTGAAATATGTGACTATATTGGATGCTTG CTATTTGTCATTTACTGTATTTCTAGCGCT TGTGCCATGTAAGGGCTGATGAACTCAAAC MGTAGCTCAGCTTTTTGCATGCTCTTTTTT ACCCACCTAGAGGTCCAGTGTTTCCTGTGA TACTTACCTCTAAACTGCTAAATAGCCACA TTTTACGGAAGCATATTCTTCATACCTTTG C | 14 |
| **UGT2B4** | rs1845556 (at position 121) | [G/A]* | A | TTACTCATTAAAATGTAACGTTTTCTAACA CCTGTAGGATGTACATCGCCCTCAGGAGTT CTTAAACAAAAGGATCAAATCACAGGGGCA GAATTTGGTGATAGCTCTTACCTGACCCCA RTATCATGATAATACCAAAACTTTACCTGC AACTCTTAATTGTATTTATTCCTGATCACA ATATTAATGCCAAATTAATTACCAATGAAA AACACCTTCTGTATGAGTTTATCATCTTCC A | 15 |
| **ABCB4** | rs1149222 (at position 121) | [A/C]* | C | TTATCTTTTTCACTTTCATGTTGTAGCCAG ACTGAGAATCTTATGATTTCTATATGGGAT GGGGGGTAGATAAATTATCGGTGGGACCAA AATTGACAGTATATTTTGCTGTGGTTCCCC MTCTCCAAAAAATTATCACTGCATGTTTGG TATGATTTTAAATAAACTACATTAAATAAA CTGACTACAGTGAAAAAGAAAAAGTTTTGA CGTAAAAAAGTAAGTCACTGCATTCAAATA T | 16 |
| **SULT1E1** | rs1910465 (at position 121) | [G/A]* | A | TGGCAGTGAGATCAGAGGTCTGATAGAGAG CAGGACATAGAAGGTGATCTGCCTGATCAG GTAATTAGTTATATACAAAAGGGGAATTAC CTGGCTGAGGTAGGGTTTCTCCACTCCAAA RTTACTCTTCCCCTCATACTGCACTTGCCC TTTATAAGAAAGTCCCAATATGGATCTCAC ACTTAAGGAATGGCACGGGGAACTATACAT AAGTAGGTGAAATTCTTCTGTATGGTATAT T | 17 |
| **UGT2B4** | rs7441743 | [G/A] | A | ACAAGATAATTAAACTAGGTAATTTTCTGA AAGAAGTTAGAATAATGTGGCAAAAATGT AGGCAAAGTGTTTGTGCCTTGAAAAAAAAT ACATATATTCRTATATAGGCATAATTTAAT TTTTATGTATTATATATTTTACCCATGTGT ATTTACAAGAAAGGCAAAGTGGTGAGAGAA TCATGAGGGAAAACTGCATCC | 18 |
| **CYP2C19** | rs10786172 | [G/A] | A | GCAGGAAGAGACAACATAAGTTGGCCTGGG ATTGCATGTTGGTTTTATTATTAAAAGGCC ATTTTGAACAGCAAGTGTATTGTTAAAGAT AATTTTTCTCRTTCTCAAAACTTCAGGTTC AAATGCTGGAGTAGGGAAAATGGAAACTCT TCTTATTGAAGGATAAATGGTAATCCTAGA AATGTAGTGAGGCTGCCTGAA | 19 |
| **ABCC3** | rs2107441 (at position 121) | [A/G] | G | GAAAACAAAAAGGTTCCCCTATTCCCTCCC CAGCAGCCCTCTCTGTGGCCTGGAGGGTTT CTCTGGAAGAGGGCAGTGCCTGGAGGGGAG CCTCTGGATAGTGATGTGGAAATATTAACA RCAGAGTCAAAGCGGGTATTTTGGGGTAGT CGGGGAGAAAACTGTGACCTCTCCCCCCAA TGATGTAAAATCTTTATCAATGATGGAAAC TGTTGTGTCTCATTAAAAGGGTTTTGAGGT G | 20 |

It will be appreciated by a person of skill in the art that further linked polymorphic sites and combined polymorphic sites may be determined. A haplotype of the above genes can be created by assessing polymorphisms in normal subjects using a program that has an expectation maximization algorithm (for example PHASE). A constructed haplotype of these genes may be used to find combinations of SNPs that are in LD with the tag SNPs (tSNPs) identified herein. Accordingly, the haplotype of an individual could be determined by genotyping other SNPs or other polymorphisms that are in LD with the tSNPs identified herein. Single polymorphic sites or combined polymorphic sites in LD may also be genotyped for assessing subject risk of cardiotoxicity following anthracycline treatment.

It will be appreciated by a person of skill in the art that the numerical designations of the positions of polymorphisms within a sequence are relative to the specific sequence and the orientation of the strand being read (i.e. forward or reverse). Also the same positions may be assigned different numerical designations depending on the way in which the sequence is numbered and the sequence chosen. Furthermore, sequence variations within the population, such as insertions or deletions, may change the relative position and subsequently the numerical designations of particular nucleotides at and around a polymorphic site. For example, the sequences represented by accession numbers NM_003786, Y 17151, BC 104952, BC050370, AF 154001 all comprise ABCC3 nucleotide sequences, but may have some sequence differences and numbering differences between them. Furthermore, one of skill in the art will appreciate that a variety of sequencing, amplification, extension, genotyping or hybridization primers or probes may be designed to specifically identify the polymorphisms described in TABLE 2, and the sequences flanking the various polymorphisms as provided herein are illustrative examples. One of skill in the art will also appreciate that a variety of sequencing, amplification, extension, genotyping or hybridization primers or probes adjacent to, complimentary to, or overlapping with the sequences provided in TABLE 2, may be developed or designed for the identification of the polymorphisms described herein, without going beyond the scope of various embodimants of the invention as described herein.

One example of a partial gene sequence is a human ABCC3 gene sequence illustrated as GenBank accession # NM_003786. The genomic sequence of the human ABCC3 gene (NC_000017.9 nucleotides 45979561-46185071) further includes 5' and 3' untranslated sequences, introns and the like. Sequence databases with this information, such as GenBank, operated by the National Centre for Biotechnology Information (NCBI) store such information in a retrievable format, and are publicly accessible. A person of skill in the art will appreciate the various methods and tools that may be used to access such information, in a context suitable to their particular application of aspects described herein

Polymorphic sites in SEQ ID NO:1-20 are identified by their variant designation (i.e. M, W, Y, S, R, K, V, B, D, H or by "-" for a deletion, a "+"or for example "G" etc. for an insertion).

An "rs" prefix designates a SNP in the database is found at the NCBI SNP database (http://www.ncbi.nlm.nih.gov/entrez/query.fcgi?db=Snp). The "rs" numbers are the NCBI| rsSNP ID form.

The Sequences given in TABLE 2 (SEQ ID NO:1-20) above may be useful to a person of skill in the art in the design of primers and probes or other oligonucleotides or PNAs for the identification of polymorphisms as described herein.

An "allele" is defined as any one or more alternative forms of a given gene. In a diploid cell or organism the members of an allelic pair (i.e. the two alleles of a given gene) occupy corresponding positions (loci) on a pair of homologous chromosomes and if these alleles are genetically identical the cell or organism is said to be "homozygous", but if genetically different the cell or organism is said to be "heterozygous" with respect to the particular gene.

A "gene" is an ordered sequence of nucleotides located in a particular position on a particular chromosome that encodes a specific functional product and may include untranslated and untranscribed sequences in proximity to the coding regions (5' and 3' to the coding sequence). Such non-coding sequences may contain regulatory sequences needed for transcription and translation of the sequence or introns etc. or may as yet to have any function attributed to them beyond the occurrence of the SNP of interest.

A "genotype" is defined as the genetic constitution of an organism, usually in respect to one gene or a few genes or a region of a gene relevant to a particular context (i.e. the genetic loci responsible for a particular phenotype).

A "phenotype" is defined as the observable characters of an organism. In gene association studies, the genetic model at a given locus can change depending on the selection pressures (i.e., the environment), the population studied, or the outcome variable (i.e., the phenotype).

A similar observation would be seen in a gene association study with the hemoblobin, beta gene (HBB) with mortality as the primary outcome variable. A mutation in the HBB gene, which normally produces the beta chain subunit of hemoglobin (B allele), results in an abnormal beta chain called hemoglobin S (S allele; Allison A (1955) Cold Spring Harbor Symp. Quant. Biol. 20:239-255). Hemoglobin S results in abnormal sickle-shaped red blood cells which lead to anemia and other serious complications including death. In the absence of malaria, a gene association study with the HBB gene would suggest a codominant model (survival(BB) > survival (BS) > survival (SS)). However, in the presence of marlaria, a gene association study with the HBB gene would suggest a heterozygote advantage model (survival(BB) < survival(BS) > survival(SS)).

A "single nucleotide polymorphism" (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. A "transition" is the replacement of one purine by another purine or one pyrimidine by another pyrimidine. A "transversion" is the replacement of a purine by a pyrimidine or vice versa. Single nucleotide polymorphisms can also arise from a deletion (represented by "-" or *"del")* of a nucleotide or an insertion (represented by "+" or *"ins"* or "I") of a nucleotide relative to a reference allele. Furthermore, a person of skill in the art would appreciate that an insertion or deletion within a given sequence could alter the relative position and therefore the position number of another polymorphism within the sequence. Furthermore, although an insertion or deletion may by some definitions not qualify as a SNP as it may involve the deletion of or insertion of more than a single nucleotide at a given position, as used herein such polymorphisms are also called SNPs as they generally result from an insertion or deletion at a single site within a given sequence.

A "subject", as used herein, refers to a patient or test subject, for example a human patient. The subject may have been previously diagnosed with a neoplastic disorder, or may be suspected of having a neoplastic disorder and thus may be a candiate for a chemotherapeutic regimen. The subject may be selected as part of a general population (for example a 'control' subject), or may be selected as part of a particular ethnic, gender, age or genetic subgroup of a population, or may be excluded from selection as part of a particular ethnic, gender, age or genetic subgroup of a population. Patients and test subjects, whether control or not, may be generally referred to as a subject.

As used herein, the terms "cancer" or "neoplastic condition" or "neoplastic disorder" or "neoplastic disease" refer to a proliferative disorder caused or characterized by the proliferation of cells which have lost susceptibility to normal growth control. A "cancer" or "neoplastic condition" or "neoplastic disorder" or "neoplastic disease" may include tumors and any other proliferative disorders. Cancers of the same tissue type usually originate in the same tissue, and may be divided into different subtypes based on their biological characteristics. Four general categories of cancers are carcinoma (epithelial tissue derived), sarcoma (connective tissue or mesodermal derived), leukemia (blood-forming tissue derived) and lymphoma (lymph tissue derived). Over 200 different types of cancers are known, and every organ and tissue of the body may be affected. Specific examples of cancers that do not limit the definition of cancer may include melanoma, leukemia, astrocytoma, glioblastoma, retinoblastoma, lymphoma, glioma, Hodgkins' lymphoma and chronic lymphocyte leukemia. Examples of organs and tissues that may be affected by various cancers include pancreas, breast, thyroid, ovary, uterus, testis, prostate, thyroid, pituitary gland, adrenal gland, kidney, stomach, esophagus or rectum, head and neck, bone, nervous system, skin, blood, nasopharyngeal tissue, lung, urinary tract, cervix, vagina, exocrine glands and endocrine glands. Alternatively, a cancer may be multicentric or of unknown primary site (CUPS).

As used herein, a "therapeutic regimen" refers to a chemotherapeutic regimen or a radiotherapy regimen, or a combination thereof.

As used herein, a "chemotherapeutic regimen" or "chemotherapy" refers to the use of at least one chemotherapy agent to destroy cancerous cells. There are a myriad of such chemotherapy agents available for treating cancer. Chemotherapy agents may be administered to a subject in a single bolus dose, or may be administered in smaller doses over time. A single chemotherapeutic agent may be used (single-agent therapy) or more than one agent may be used in combination (combination therapy). Chemotherapy may be used alone to treat some types of cancer. Alternatively, chemotherapy may be used in combination with other types of treatment, for example, radiotherapy or alternative therapies (for example immunotherapy) as described herein. Additionally, a chemosensitizer may be administered as a combination therapy with a chemotherapy agent.

As used herein, a "chemotherapeutic agent" or "chemotherapeutic agent" refers to a medicament that may be used to treat cancer, and generally has the ability to kill cancerous cells directly. Examples of chemotherapeutic agents include alkylating agents, antimetabolites, natural products, hormones and antagonists, and miscellaneous agents. Examples of alternate names are indicated in brackets. Examples of alkylating agents include nitrogen mustards such as mechlorethamine, cyclophosphamide, ifosfamide, melphalan (L-sarcolysin) and chlorambucil; ethylenimines and methylmelamines such as hexamethylmelamine and thiotepa; alkyl sulfonates such as busulfan; nitrosoureas such as carmustine (BCNU), semustine (methyl-CCNU), lomustine (CCNU) and streptozocin (streptozotocin); DNA synthesis antagonists such as estramustine phosphate; and triazines such as dacarbazine (DTIC, dimethyl-triazenoimidazolecarboxamide) and temozolomide . Examples of antimetabolites include folic acid analogs such as methotrexate (amethopterin); pyrimidine analogs such as fluorouracin (5-fluorouracil, 5-FU, 5FU), floxuridine (fluorodeoxyuridine, FUdR), cytarabine (cytosine arabinoside) and gemcitabine; purine analogs such as mercaptopurine (6-mercaptopurine, 6-MP), thioguanine (6-thioguanine, TG) and pentostatin (2'-deoxycoformycin, deoxycoformycin), cladribine and fludarabine; and topoisomerase inhibitors such as amsacrine. Examples of natural products include vinca alkaloids such as vinblastine (VLB) and vincristine; taxanes such as paclitaxel and docetaxel (Taxotere); epipodophyllotoxins such as etoposide and teniposide; camptothecins such as topotecan or irinotecan; antibiotics such as dactinomycin (actinomycin D), bleomycin, mitomycin (mitomycin C); anthracycline antibiotics such as daunorubicin (daunomycin, rubidomycin), doxorubicin, idarubicin, epirubicin; enzymes such as L-asparaginase; and biological response modifiers such as interferon alpha and interleukin 2. Examples of hormones and antagonists include luteinising releasing hormone agonists such as buserelin; adrenocorticosteroids such as prednisone and related preparations; progestins such as hydroxyprogesterone caproate, medroxyprogesterone acetate and megestrol acetate; estrogens such as diethylstilbestrol and ethinyl estradiol and related preparations; estrogen antagonists such as tamoxifen and anastrozole; androgens such as testosterone propionate and fluoxymesterone and related preparations; androgen antagonists such as flutamide and bicalutamide; and gonadotropin-releasing hormone analogs such as leuprolide. Examples of miscellaneous agents include thalidomide; platinum coordination complexes such as cisplatin (cis-DDP), carboplatin, oxaliplatin, tetraplatin, ormiplatin, iproplatin or satraplatin; anthracenediones such as mitoxantrone; substituted ureas such as hydroxyurea; methylhydrazine derivatives such as procarbazine (N-methylhydrazine, MIH); adrenocortical suppressants such as mitotane (o,p'-DDD) and aminoglutethimide; RXR agonists such as bexarotene; or tyrosine kinase inhibitors such as imatinib. Alternate names and trade-names of these and additional examples of chemotherapeutic agents, and their methods of use including dosing and administration regimens, will be known to an individual versed in the art, and may be found in, for example "The Pharmacological basis of therapeutics", 10th edition. HARDMAN HG., LIMBIRD LE. editors. McGraw-Hill, New York, or in "Clinical Oncology", 3rd edition. Churchill Livingstone/ Elsevier Press, 2004. ABELOFF, MD. editor.

### 2. General Methods

Once a subject is identified as a candidate for anthracycline administration, then genetic sequence information may be obtained from the subject to determine the risk of cardiotoxicity for the subject. Genetic sequence information may be obtained from a subject by any of several methods. For example, a biological sample comprising genetic material with a sequence or sequences of interest, may be obtained from the subject, for example a blood sample, a saliva sample, a hair sample including a follicle, skin scraping, such as a cheek scraping and the like. Or alternatively genetic sequence information may already have been obtained from the subject. For example, a subject may have already provided a biological sample for other purposes or may have even had their genetic sequence determined in whole or in part and stored for future use. Genetic sequence information may be obtained in numerous different ways and may involve the collection of a biological sample that contains genetic material, particularly, genetic material containing the sequence or sequences of interest. Many methods are known in the art for collecting biological samples and extracting genetic material from those samples. Genetic material can be extracted from blood, tissue, hair and other biological material. There are many methods known to isolate DNA and RNA from biological material. Typically, DNA may be isolated from a biological sample when first the sample is lysed and then the DNA is separated from the lysate according to any one of a variety of multi-step protocols, which can take varying lengths of time. DNA isolation methods may involve the use of phenol (Sambrook, J. et al., "Molecular Cloning", Vol. 2, pp. 9.14-9.23, Cold Spring Harbor Laboratory Press (1989) and Ausubel, Frederick M. et al., "Current Protocols in Molecular Biology", Vol. 1, pp. 2.2.1-2.4.5, John Wiley & Sons, Inc. (1994)). Typically, a biological sample is lysed in a detergent solution and the protein component of the lysate is digested with proteinase for 12-18 hours. Next, the lysate is extracted with phenol to remove most of the cellular components, and the remaining aqueous phase is processed further to isolate DNA. In another method, described in Van Ness et al. (U.S. Pat. # 5,130,423), non-corrosive phenol derivatives are used for the isolation of nucleic acids. The resulting preparation is a mix of RNA and DNA.

Other methods for DNA isolation utilize non-corrosive chaotropic agents. These methods, which are based on the use of guanidine salts, urea and sodium iodide, involve lysis of a biological sample in a chaotropic aqueous solution and subsequent precipitation of the crude DNA fraction with a lower alcohol. The resulting nucleic acid sample may be used 'as-is' in further analyses or may be purified further. Additional purification of the precipitated, crude DNA fraction may be achieved by any one of several methods, including, for example, column chromatography (Analects, (1994) Vol 22, No. 4, Pharmacia Biotech), or exposure of the crude DNA to a polyanion-containing protein as described in Koller (U.S. Pat. # 5,128,247).

Yet another method of DNA isolation, which is described by Botwell, D. D. L. (Anal. Biochem. (1987) 162:463-465) involves lysing cells in 6M guanidine hydrochloride, precipitating DNA from the lysate at acid pH by adding 2.5 volumes of ethanol, and washing the DNA with ethanol. Numerous other methods are known in the art to isolate both RNA and DNA, such as the one described by CHOMCZYNSKI (U.S. Pat. # 5,945,515), whereby genetic material can be extracted efficiently in as little as twenty minutes. EVANS and HUGH (U.S. Pat. # 5,989,431) describe methods for isolating DNA using a hollow membrane filter.

The level of expression of specific nucleic acids such as mRNAs or microRNAs, copy number of a gene, or the degree of heterozygosity for a polymorphism may also be determined once the nucleic acid sample has been obtained. Quantitative and semi-quantitative methods are known in the art, and may be found in, for example AUSUBEL, supra; SAMBROOK, supra or Harrison's Principles of Internal Medicine 15th ed. BRAUNWALD et al eds. McGraw-Hill.

Once a subject's genetic material has been obtained from the subject it may then be further be amplified by Reverse Transcription Polymerase Chain Reaction (RT-PCR), Polymerase Chain Reaction (PCR), Transcription Mediated Amplification (TMA), Ligase chain reaction (LCR), Nucleic Acid Sequence Based Amplification (NASBA) or other methods known in the art, and then further analyzed to detect or determine the presence or absence of one or more polymorphisms or mutations in the sequence of interest, provided that the genetic material obtained contains the sequence of interest. Particularly, a person may be interested in determining the presence or absence of a polymorphism in a cardiotoxicity associated gene sequence, as described herein.

Detection or determination of a nucleotide identity, or the presence of one or more single nucleotide polymorphism(s) (SNP typing), may be accomplished by any one of a number methods or assays known in the art. Many DNA typing methodologies are useful for use in the detection of SNPs. The majority of SNP genotyping reactions or assays can be assigned to one of four broad groups (sequence-specific hybridization, primer extension, oligonucleotide ligation and invasive cleavage). Furthermore, there are numerous methods for analyzing/detecting the products of each type of reaction (for example, fluorescence, luminescence, mass measurement, electrophoresis, etc.). Furthermore, reactions can occur in solution or on a solid support such as a glass slide, a chip, a bead, etc.

In general, sequence-specific hybridization involves a hybridization probe, which is capable of distinguishing between two DNA targets differing at one nucleotide position by hybridization. Usually probes are designed with the polymorphic base in a central position in the probe sequence, whereby under optimized assay conditions only the perfectly matched probe target hybrids are stable and hybrids with a one base mismatch are unstable. A strategy which couples detection and sequence discrimination is the use of a "molecular beacon", whereby the hybridization probe (molecular beacon) has 3' and 5' reporter and quencher molecules and 3' and 5' sequences which are complementary such that absent an adequate binding target for the intervening sequence the probe will form a hairpin loop. The hairpin loop keeps the reporter and quencher in close proximity resulting in quenching of the fluorophor (reporter) which reduces fluorescence emissions. However, when the molecular beacon hybridizes to the target the fluorophor and the quencher are sufficiently separated to allow fluorescence to be emitted from the fluorophor.

Similarly, primer extension reactions (i.e. mini sequencing, nucleotide-specific extensions, or simple PCR amplification) are useful in sequence discrimination reactions. For example, in mini sequencing a primer anneals to its target DNA immediately upstream of the SNP and is extended with a single nucleotide complementary to the polymorphic site. Where the nucleotide is not complementary, no extension occurs.

Oligonucleotide ligation assays require two sequence-specific probes and one common ligation probe per SNP. The common ligation probe hybridizes adjacent to a sequence-specific probe and when there is a perfect match of the appropriate sequence-specific probe, the ligase joins both the sequence-specific and the common probes. Where there is not a perfect match the ligase is unable to join the sequence-specific and common probes. Probes used in hybridization can include double-stranded DNA, single-stranded DNA and RNA oligonucleotides, and peptide nucleic acids. Hybridization methods for the identification of single nucleotide polymorphisms or other mutations involving a few nucleotides are described in the U.S. Pat. 6,270,961; 6,025,136; and 6,872,530. Suitable hybridization probes for use in accordance with the invention include oligonucleotides and PNAs from about 10 to about 400 nucleotides, alternatively from about 20 to about 200 nucleotides, or from about 30 to about 100 nucleotides in length.

A unimolecular segment amplification method for amplifying nucleic acids is described in US patent 5854033. A rolling circle replication reporter system may be used for identification of polymorphisms or mutations.

An invasive cleavage method employs an "Invader™" (Applied Biosystems) probe and sequence-specific probes to hybridize with the target nucleic acid, usually DNA, with an overlap of one nucleotide. When the sequence specific probe is an exact match to the site of polymorphism, the overlapping probes form a structure that is specifically cleaved by a FLAP endonuclease, Release of the 5' end of the allele-specific probe may be detected by known methods as described. See for example, Lu, M., et al. J. Am. Chem. Soc. 2001, 124, 7924 - 7931; Lyamichev, et al. 1999. Nature Biotech. 17, 292 - 296; Landegren et al. 1998. Genome Research, 8, 769 - 776; Brookes, 1999. Gene 234, 177 - 186; Chen, et al 2004. J. Am. Chem. Soc. 126, 3016-3017; Wang, D.G., et al. Science 1998, 280, 1077 - 1082. The TaqMan™ assay (Applied Biosystems) exploits the 5' exonuclease activity of the Taq polymerase to displace and cleave an oligonucleotide probe hybridized to the target nucleic acid, usually DNA, generating a fluorescent signal. See, for example U.S. Patents 4,683,202, 4,683,195, and 4,965,188.

5' exonuclease activity or TaqMan™ assay (Applied Biosystems) is based on the 5' nuclease activity of Taq polymerase that displaces and cleaves the oligonucleotide probes hybridized to the target DNA generating a fluorescent signal. It is necessary to have two probes that differ at the polymorphic site wherein one probe is complementary to the 'normal' sequence and the other to the mutation of interest. These probes have different fluorescent dyes attached to the 5' end and a quencher attached to the 3' end when the probes are intact the quencher interacts with the fluorophor by fluorescence resonance energy transfer (FRET) to quench the fluorescence of the probe. During the PCR annealing step the hybridization probes hybridize to target DNA. In the extension step the 5' fluorescent dye is cleaved by the 5' nuclease activity of Taq polymerase, leading to an increase in fluorescence of the reporter dye. Mismatched probes are displaced without fragmentation. The presence of a mutation in a sample is determined by measuring the signal intensity of the two different dyes.

The Illumina Golden Gate™ Assay uses a combined oligonucleotide ligation assay/ allele-specific hybridization approach (SHEN R et al Mutat Res 2005573:70-82*).* The first series of steps involve the hybridization of three oligonucleotides to a set of specific target SNPs; two of these are fluorescently-labelled allele-specific oligonucleotides (ASOs) and the third a locus-specific oligonucleotide (LSO) binding 1-20 bp downstream of the ASOs. A second series of steps involve the use of a stringent polymerase with high 3' specificity that extends only oligonucleotides specifically matching an allele at a target SNP. The polymerase extends until it reaches the LSO. Locus-specificity is ensured by requiring the hybridization of both the ASO and LSO in order that extension can proceed. After PCR amplification with universal primers, these allele-specific oligonucleotide extension products are hybridized to an array which has multiple discretely tagged addresses (in this case 1536 addresses) which match an address embedded in each LSO. Fluorescent signals produced by each hybridization product are detected by a bead array reader from which genotypes at each SNP locus may be ascertained.

It will be appreciated that numerous other methods for sequence discrimination and detection are known in the art and some of which are described in further detail below. It will also be appreciated that reactions such as arrayed primer extension mini sequencing, tag microarrays and sequence-specific extension could be performed on a microarray. One such array based genotyping platform is the microsphere based tag-it high throughput genotyping array (BORTOLIN S. et al. Clinical Chemistry (2004) 50(11): 2028-36). This method amplifies genomic DNA by PCR followed by sequence-specific primer extension with universally tagged genotyping primers. The products are then sorted on a Tag-It array and detected using the Luminex xMAP system.

Mutation detection methods may include but are not limited to the following: Restriction Fragment Length Polymorphism (RFLP) strategy - An RFLP gel-based analysis can be used to indicate the presence or absence of a specific mutation at polymorphic sites within a gene. Briefly, a short segment of DNA (typically several hundred base pairs) is amplified by PCR. Where possible, a specific restriction endonuclease is chosen that cuts the short DNA segment when one polymorphism is present but does not cut the short DNA segment when the polymorphism is not present, or vice versa. After incubation of the PCR amplified DNA with this restriction endonuclease, the reaction products are then separated using gel electrophoresis. Thus, when the gel is examined the appearance of two lower molecular weight bands (lower molecular weight molecules travel farther down the gel during electrophoresis) indicates that the DNA sample had a polymorphism was present that permitted cleavage by the specific restriction endonuclease. In contrast, if only one higher molecular weight band is observed (at the molecular weight of the PCR product) then the initial DNA sample had the polymorphism that could not be cleaved by the chosen restriction endonuclease. Finally, if both the higher molecular weight band and the two lower molecular weight bands are visible then the DNA sample contained both polymorphisms, and therefore the DNA sample, and by extension the subject providing the DNA sample, was heterozygous for this polymorphism;

For example the Maxam-Gilbert technique for sequencing (MAXAM AM. and GILBERT W. Proc. Natl. Acad. Sci. USA (1977) 74(4):560-564) involves the specific chemical cleavage of terminally labelled DNA. In this technique four samples of the same labeled DNA are each subjected to a different chemical reaction to effect preferential cleavage of the DNA molecule at one or two nucleotides of a specific base identity. The conditions are adjusted to obtain only partial cleavage, DNA fragments are thus generated in each sample whose lengths are dependent upon the position within the DNA base sequence of the nucleotide(s) which are subject to such cleavage. After partial cleavage is performed, each sample contains DNA fragments of different lengths, each of which ends with the same one or two of the four nucleotides. In particular, in one sample each fragment ends with a C, in another sample each fragment ends with a C or a T, in a third sample each ends with a G, and in a fourth sample each ends with an A or a G. When the products of these four reactions are resolved by size, by electrophoresis on a polyacrylamide gel, the DNA sequence can be read from the pattern of radioactive bands. This technique permits the sequencing of at least 100 bases from the point of labeling. Another method is the dideoxy method of sequencing was published by SANGER et al. (Proc. Natl. Acad. Sci. USA (1977) 74(12):5463-5467). The Sanger method relies on enzymatic activity of a DNA polymerase to synthesize sequence-dependent fragments of various lengths. The lengths of the fragments are determined by the random incorporation of dideoxynucleotide base-specific terminators. These fragments can then be separated in a gel as in the Maxam-Gilbert procedure, visualized, and the sequence determined. Numerous improvements have been made to refine the above methods and to automate the sequencing procedures. Similarly, RNA sequencing methods are also known. For example, reverse transcriptase with dideoxynucleotides have been used to sequence encephalomyocarditis virus RNA (ZIMMERN D. and KAESBERG P. Proc. Natl. Acad. Sci. USA (1978) 75(9):4257-4261). MILLS DR. and KRAMER FR. (Proc. Natl. Acad. Sci. USA (1979) 76(5):2232-2235) describe the use of Qβ replicase and the nucleotide analog inosine for sequencing RNA in a chain-termination mechanism. Direct chemical methods for sequencing RNA are also known (PEATTIE DA. Proc. Natl. Acad. Sci. USA (1979) 76(4):1760-1764). Other methods include those of Donis-Keller et al. (1977, Nucl. Acids Res. 4:2527-2538), SIMONCSITS A. et al. (Nature (1977) 269(5631):833-836), AXELROD VD. et al. (Nucl. Acids Res.(1978) 5(10):3549-3563), and KRAMER FR. and MILLS DR. (Proc. Natl. Acad. Sci. USA (1978) 75(11):5334-5338). Nucleic acid sequences can also be read by stimulating the natural fluoresce of a cleaved nucleotide with a laser while the single nucleotide is contained in a fluorescence enhancing matrix (U.S. Pat. # 5,674,743); In a mini sequencing reaction, a primer that anneals to target DNA adjacent to a SNP is extended by DNA polymerase with a single nucleotide that is complementary to the polymorphic site. This method is based on the high accuracy of nucleotide incorporation by DNA polymerases. There are different technologies for analyzing the primer extension products. For example, the use of labeled or unlabeled nucleotides, ddNTP combined with dNTP or only ddNTP in the mini sequencing reaction depends on the method chosen for detecting the products;

Probes used in hybridization can include double-stranded DNA, single-stranded DNA and RNA oligonucleotides, and peptide nucleic acids. Hybridization methods for the identification of single nucleotide polymorphisms or other mutations involving a few nucleotides are described in the U.S. Pat. 6,270,961; 6,025,136; and 6,872,530. Suitable hybridization probes for use in accordance with the invention include oligonucleotides and PNAs from about 10 to about 400 nucleotides, alternatively from about 20 to about 200 nucleotides, or from about 30 to about 100 nucleotides in length.

A template-directed dye-terminator incorporation with fluorescent polarization-detection (TDI-FP) method is described by FREEMAN BD. et al. (J Mol Diagnostics (2002) 4(4):209-215) for large scale screening;
Oligonucleotide ligation assay (OLA) is based on ligation of probe and detector oligonucleotides annealed to a polymerase chain reaction amplicon strand with detection by an enzyme immunoassay (VILLAHERMOSA ML. J Hum Virol (2001) 4(5):238-48; ROMPPANEN EL. Scand J Clin Lab Invest (2001) 61 (2):123-9; IANNONE MA. et al. Cytometry (2000) 39(2):131-40);
Ligation-Rolling Circle Amplification (L-RCA) has also been successfully used for genotyping single nucleotide polymorphisms as described in QI X. et al. Nucleic Acids Res (2001) 29(22):E116;
5' nuclease assay has also been successfully used for genotyping single nucleotide polymorphisms (AYDIN A. et al. Biotechniques (2001) (4):920-2, 924, 926-8.);
Polymerase proofreading methods are used to determine SNPs identities, as described in WO 0181631;
Detection of single base pair DNA mutations by enzyme-amplified electronic transduction is described in PATOLSKY F et al. Nat Biotech. (2001) 19(3):253-257;
Gene chip or microarray technologies are also known for single nucleotide polymorphism discrimination whereby numerous polymorphisms may be tested for simultaneously on a single array (for example: EP 1120646; and GILLES PN. et al. Nat. Biotechnology (1999) 17(4):365-70);

Matrix assisted laser desorption ionization time of flight (MALDI-TOF) mass spectroscopy is also useful in the genotyping single nucleotide polymorphisms through the analysis of microsequencing products (HAFF LA. and SMIRNOV IP. Nucleic Acids Res. (1997) 25(18):3749-50; HAFF LA. and SMIRNOV IP. Genome Res. (1997) 7:378-388; SUN X. et al. Nucleic Acids Res. (2000) 28 e68; BRAUN A. et al. Clin. Chem. (1997) 43:1151-1158; LITTLE DP. et al. Eur. J. Clin. Chem. Clin. Biochem. (1997) 35:545-548; FEI Z. et al. Nucleic Acids Res. (2000) 26:2827-2828; and BLONDAL T. et al. Nucleic Acids Res. (2003) 31 (24):e 155).

Sequence-specific PCR methods have also been successfully used for genotyping single nucleotide polymorphisms (HAWKINS JR. et al. Hum Mutat (2002) 19(5):543-553). Alternatively, a Single-Stranded Conformational Polymorphism (SSCP) assay or a Cleavase Fragment Length Polymorphism (CFLP) assay may be used to detect mutations as described herein.

US 7,074,597 describes methods for multiplex genotyping using solid phase capturable dideoxynucleotides and mass spectrometry. Nucleotide identity is detected at a specific site of a nucleic acid sample by contacting DNA-primer complex with labeled dideoxynucleotides (ddNTPs) to generate labeled single base extended (SBE) primer. The identifying ddNTP may be within the SBE primer.

Multiplex analysis of PCR-amplified products may also be used to detect specific SNPs. Reporting DNA sequences comprising a fluorophore on a 5' end may be used to combine a multiplex PCR amplification reaction with microsphere based hybridization (US 7,083,951). Other multiplex detection methods include BeadArray™ and similar hybridization-based methods, for example, those described in US Patent Nos. 6,429,027, 6,396,995, 6,355,431.

Microarray or 'gene chips' of oligonucleotides may be used for SNP discrimination. Oligonucleotides may be nucleic acids or modified nucleic acids, including PNAs, and may be 'spotted' onto a solid matrix, such as a glass or plastic slide. Alternatively, oligonucleotides may be synthesized in situ on the slide. See, for example, GAO et al 2004. Biopolymers 73:579-596; US 5,445,934; US 5,744,305, US 5,800,992, US 5,796,715.

Alternatively, if a subject's sequence data is already known, then obtaining may involve retrieval of the subjects nucleic acid sequence data (for example from a database), followed by determining or detecting the identity of a nucleic acid or genotype at a polymorphic site by reading the subject's nucleic acid sequence at the one or more polymorphic sites.

Once the identity of a polymorphism(s) is determined or detected an indication may be obtained as to the subject's risk of cardiotoxicity following anthracycline administration. Methods for predicting a subject's risk of cardiotoxicity following anthracycline administration may be useful in making decisions regarding the administration of anthracycline(s).

### TREATMENT

Anthracycline compounds (for example, doxorubicin) may be used to treat a variety of cancers in children and adults. In a given therapeutic regimen, the anthracycline compound may be administered alone or in combination with other chemotherapeutic agents in various doses and compositions, depending on the type of cancer, age of subject, health of subject, body mass, etc. The choice of dose, chemotherapeutic agents or combinations, methods of administration and the like will be known to those skilled in the art. Further, methods of assessing response to treatment and side effects are also known. For example, heart function in a subject suspected of experiencing cardiotoxicity may be assessed by various methods including medical history, electrocardiogram (ECG) monitoring, endomyocardial biopsy, radionuclide angiography (MUGA scan) or LVEF monitoring with serial echo or exercise stress testing, or other methods that may be dependent on the age and condition of the subject, as are known in the art. Early signs of cardiotoxicity may include persistent reduction in the voltage of the QRS wave, prolongation of the systolic time interval, or reduction of LVEF as determined by echo or MUGA. A reduction of 10% to below the lower limit of normal, 20% at any level, or an absolute LVEF ≤45% indicates deterioration of cardiac function.)

Response to a therapeutic regimen may be monitored. Tumor staging provides a method to assess the size and spread of a tumor in response to a treatment regimen. The TNM tumor staging system uses three components to express the anatomic extent of disease: T is a measure of the local extent of tumor spread (size), N indicates the presence or absence of metastatic spread to regional lymph nodes, and M specifies the presence or absence of metastatic spread to distant sites. The combination of these classifications combine to provide a stage grouping. Clinical TNM (cTNM) defines the tumor based on clinical evidence. Pathologic TNM (pTNM) defines the tumor based on examination of a surgically resected specimen.

Changes in tumor size may be observed by various imaging methods known to physicians or surgeons in the field of oncology therapy and diagnostics. Examples of imaging methods include positron emission tomography (PET) scanning, computed tomography (CT) scanning, PET/CT scanning, magnetic resonance imaging (MRI), chemical shift imaging, radiography, bone-scan, mammography, fiberoptic colonoscopy or ultrasound. Contrast agents, tracers and other specialized techniques may also be employed to image specific types of cancers, or for particular organs or tissues, and will be known to those skilled in the art. Changes in rate of metastasis may also be observed by the various imaging methods, considering particularly the appearance, or frequency of appearance, of tumors distal to the primary site. Alternatively, the presence of tumor cells in lymph nodes adjacent and distal to the primary tumor site may also be detected and used to monitor metastasis.

A subject may be tested for a cardiotoxicity-associated polymorphism before undergoing a therapeutic regimen involving an anthracycline compound. If a subject's genotype includes a cardiotoxicity-associated polymorphism, this may indicate that the subject is at a risk for cardiotoxicity when an anthracycline compound is administered.

A subject at risk for cardiotoxicity may be administered a therapeutic regimen involving an anthracycline compound and the cardiac function monitored as described. If a decrease in cardiac function is identified, the therapeutic regimen may be altered to decrease the dose of the anthracycline compound, eliminate the dose of the anthracycline compound, or increase the dose of a second chemotherapeutic agent in the therapeutic regimen. Examples of chemotherapeutic agents that may be used in combination with an anthracycline compound in a therapeutic regimen may include, for example, cyclophosphamide, Ifosphamide, fluorouracil, Paclitaxel, vincristine, cisplatin, streptozocin, docetaxel, and the like.

A subject at risk for cardiotoxicity may also be administered a therapeutic regimen involving an anthracycline compound and the cardiac function monitored as described. The therapeutic regimen may be supplemented to include a cardioprotective agent. Examples of cardioprotective agents are known in the art, and may include those described by Wouters et al 2005. Br. J Hematol 131:561-578). For example, Dexrazoxane is a cardioprotective agent and is approved for use in conjunction with doxorubicin to reduce the incidence and severity of cardiomyopathy associated with doxorubicin administration.

Alternatively, a subject at risk for cardiotoxicity may be administered a therapeutic regimen that does not involve an anthracycline compound and the cardiac function monitored as described.

### GENES

Numerous genes are known to be involved in ADME (absorption, distribution, metabolism and elimination), for example SULT4A1, ADH1B/C, CYP2C8, NFAT5, UGT1A6/7/8/9/10, TBXAS1, ABCC1, CYP11A1, CHST3, CYP11B1, NCF4, DHFR, UGT2B4, ABCB4, SULT1E1, CYP2C19 or ABCC3. Detailed information relating to the sequence, expression patterns, molecular biology, etc of these and related genes in both Homo sapiens and in other model species is known, and may be found at, for example Entrez Gene (http://www.ncbi.nlm.nih.gov) and references therein.

Sulfotransferase family 4A, member [Homo sapiens] (SULT4A1) (alternate designations include OTTHUMP00000028875; brain sulphotransferase-like; nervous system cytosolic sulfotransferase; sulfotransferase-related protein; BR-STL-1; BRSTL 1; DJ388M5.3; MGC40032; NST; SULTX3; hBR-STL-1) maps to chromosome 22q13.2-q13.31 (about nucleotides 42551720-42589711 of Build 36.1). Representative human SULT4A1 sequences may be found in GenBank under accession numbers NM_014351, CR456588, BC030665, AL590119, AF251263, AF276342. SULT4A1 encodes a brain-specific sulfotransferase that may have a role in metabolism of neurotransmitters.

Alcohol dehydrogenase IB (class I), beta polypeptide [Homo sapiens] (ADH1B) (alternate designations include ADH, beta subunit; alcohol dehydrogenase 1B (class I), beta polypeptide; alcohol dehydrogenase 2; alcohol dehydrogenase 2 (class I), beta polypeptide; aldehyde reductase; ADH2) and alcohol dehydrogenase 1C (class I), gamma polypeptide [Homo sapiens] (ADH1C) (alternate designations include ADH3; ADH, gamma subunit; alcohol dehydrogenase 3; alcohol dehydrogenase 3 (class I), gamma polypeptide; aldehyde reductase; class I alcohol dehydrogenase, gamma subunit) map to chromosome 4q21-q23. ADH1B is found in the region comprising nucleotides 100446552-100461581 of Build 36.1; ADH1C is found in the region comprising nucleotides 100476672-100492940. Examples of nucleic acid sequences comprising ADH1B include NM_000668, X03350, M24317, BC033002. Examples of nucleic acid sequences comprising ADH1C include NM_00669, X04350, M12272, BC074786. ADH1B and ADH1C encode subunits of alcohol dehydrogenase class 1, and have a role in the metabolism of a variety of substrates including alcohol, some steroids, and some lipid peroxidation products.

Cytochrome P450, family 2, subfamily C, polypeptide 8 [Homo sapiens] (CYP2C8) (alternate designations include P450 form 1; cytochrome P450, subfamily IIC (mephenytoin 4-hydroxylase), polypeptide 8; flavoprotein-linked monooxygenase; microsomal monooxygenase; s-mephenytoin 4-hydroxylase; xenobiotic monooxygenase; CPC8; P450 MP-12/MP-20) maps to chromosome 10q23.33 (about nucleotides 96512453-97040771 of Build 36.1). Examples of nucleic acid sequences comprising TEXAS include those found in GenBank under accession numbers NM_000770, Y00498, X51535, M21942, M 17398, BC020596. CYP2C8 encodes a member of the cytochrome P450 superfamily of enzymes. CYP2C8 localizes to the endoplasmic reticulum and has a role in the metabolism of xenobiotics.

Nuclear factor of activated T-cells 5, tonicity-responsive [Homo sapiens] (NFAT5) (alternate designations include KIAA0827; NF-AT5; NFATL1; NFATZ; OREBP; TONEBP; NFAT-like protein 1; T cell transcription factor NFAT5; TonE-binding protein; glutamine rich protein H65; nuclear factor of activated T-cells 5; osmotic response element-binding protein; tonicity-responsive enhancer binding protein) maps to chromosome 16q22.1 (about nucleotides 68156498-68296054 of Build 36.1). Examples of nucleic acid sequences comprising NFAT5 include those found in GenBank under accession numbers NM_138714, NM_138713, NM_173214, NM_006599, NM_173215, Z97016, U80231, AJ243299, AF346509. NFAT5 is a transcription factor that has a role in the regulation of gene expression induced by osmotic cells.

Thromboxane A synthase 1 (platelet, cytochrome P450, family 5, subfamily A) [Homo sapiens] (TBXAS1) (alternate designations include CYP5; CYP5A1; THAS; TS; TXAS; TXS; TXA synthase; thromboxane A synthase 1 (platelet, cytochrome P450, subfamily V)) maps to chromosome 7q34-q35 (about nucleotides 139175421-139366471 of Build 36.1). Examples of nucleic acid sequences comprising TBXAS 1 include those found in GenBank under accession numbers NM_001061, NM_030984, M80647, M80646, BC041157, AK223466. TBXAS1 is a member of the cytochrome P450 superfamily. TBXAS1 is an endoplasmic reticulum membrane protein, and catalyzes the conversion of prostaglandin H2 to thromboxane A2.

ATP-binding cassette, sub-family C (CFTR/MRP), member 1 [Homo sapiens] (ABCC1) (alternate designations include ABC29; ABCC; DKFZp686N04233; DKFZp781G125; GS-X; MRP; MRP1; ATP-binding cassette, sub-family C, member 1; multidrug resistance protein; multiple drug resistance protein 1; multiple drug resistance-associated protein) maps to chromosome 16p13.1 (about nucleotides 15950935-16143774 of Build 36.1). Examples of nucleic acid sequences comprising ABCC include those found in GenBank under accession numbers NM_004996, NM_019900, NM_019898, NM_019899, NM_019902, NM_019901, NM_019862. ABCC1 is a member of the superfamily of AT-binding cassette transporters. ABCC1 functions as a multispecific organic anion transporter, with a variety of substrates. ABCC1 also has a role in transport of glucuronides and sulphate conjugates of steroid homes and bile salts.

Cytochrome P450, family 11, subfamily A, polypeptide 1 [Homo sapiens] (CYP11A1) (alternate designations include CYP11A; P450SCC; cholesterol 20-22 desmolase; cholesterol monooxygenase (side-chain cleaving); cytochrome P450, subfamily XIA; cytochrome P450, subfamily XIA (cholesterol side chain cleavage); cytochrome P450C11A1; steroid 20-22-lyase) maps to chromosome 15q23-q24 (about nucleotides 72417157-72447020 of Build 36.1). Examples of nucleic acid sequences comprising CYP11A1 include those found in GenBank under accession numbers NM_000781, M28253, M14565, BC032329. CYP11A1 is a member of the cytochrome P450 superfamily. CYP11A1 is localized to the mitochondrial inner membrane and catalyzes the conversion of cholesterol to some steroid hormones.

Carbohydrate (chondroitin 6) sulfotransferase 3 [Homo sapiens] (CHST3) (alternate designations include chondroitin 6-sulfotransferase; C6ST; C6ST1) maps to chromosome 10q22.1 (about nucleotides 73394126-73443318 of Build 36.1). Examples of nucleic acid sequences comprising CHST3 include those found in GenBank under accession numbers NM_004273, BC104856, BC093690, AB017915, AB012192. CHST3 catalyzes the transfer of a sulphate group from a sulphated nucleotide donor (PAPS) to a GalNAc residue in the formation of chondroitin sulphate.

Cytochrome P450, family 11, subfamily B, polypeptide 1 [Homo sapiens] (CYP11B1) (alternate designations include P450, subfamily XIB (steroid 11-beta-hydroxylase), polypeptide 1; cytochrome p450 XIB1; steroid 11-beta-hydroxylase; steroid 11-beta-monooxygenase; CPN1; CYP11B; DKFZp686B05283; FHI; FLJ36771; P450C11) maps to chromosome 8q21 (about nucleotides 14390775-143958238 of Build 36.1). Examples of nucleic acid sequences comprising CYP11B include those found in GenBank under accession numbers NM_00497, NM_001026213, X55764, M24667, BC096287, AK094090, AF478474. CYP11B is a member of the cytochrome P450 superfamily. CYP11B1 localizes to the mitochondrial inner membrane and is involved in conversion of some steroids to cortisol.

Neutrophil cytosolic factor 4, 40kDa [Homo sapiens] (NCF4) (alternate designations include CTA-833B7.1; MGC3810; NCF; P40PHOX; SH3PXD4; OTTHUMP00000028736; OTTHUMP00000043756; neutrophil NADPH oxidase factor 4; neutrophil cytosol factor 4; neutrophil cytosolic factor 4 (40kD)) maps to chromosome 22q13.1 (about nucleotides 35586991-35604005 of Build 36.1). Examples of nucleic acid sequences comprising NCF4 include those found in GenBank under accession numbers NM_013416, NM_000631, X77094, CR542078, BT007346, AK223324, AB025219. NCF4 is a cytosolic regulatory component of NADPH-oxidase.

Dihydrofolate reductase [Homo sapiens| (DHFR) maps to chromosome 5q11.2-q13.2 (about nucleotides 79957801-79986556 of Build 36.1). Examples of nucleic acid sequences comprising DHFR include those found in GenBank under accession numbers TNM_000791, V00507, J00140, BC071996, BC000192, BC003584. DHFR converts dihydrofolate into tetrahydrofolate (THF). THF is a methyl donor involved in the de novo synthesis of some nucleotides and amino acids.

Sulfotransferase family 1E, estrogen-preferring, member 1 [Homo sapiens] (SULT1E1) (alternate designations include EST; EST-1; MGC34459; STE; estrogen sulfotransferase; estrone sulfotransferase; sulfotransferase, estrogen-preferring) maps to chromosome 4q13.1 (about nucleotides 70741519-70760459 of Build 36.1). Examples of nucleic acid sequences comprising SULT1E1 include those found in GenBank under accession numbers NM_005420, Y11195, U55764, U08098, CR407621. Sulfotransferase enzymes catalyze the sulphate conjugation of various indigenous and xenobiotic compounds.

UDP glucuronosyltransferase 2 family, polypeptide B4 [Homo sapiens] (UGT2B4) (alternate designations include UBG2B11; UDP glycosyltransferase 2 family, polypeptide B4; UDP-glucuronyltransferase, family 2, beta-4) maps to chromosome 4Q13 (about nucleotides 70380473-70396205 of Build 36.1). Examples of nucleic acid sequences comprising UGT2B4 include those found in GenBank under accession numbers NM_021139, Y00317, BC0260264, AY529122, AJ005162. UDP-glucuronosyltransferases are a group of isoenzymes located in the hepatic endoplasmic reticulum. UGT2 family may have a role in glucuronidation of steroid or bile acids.

Cytochrome P450, family 2, subfamily C, polypeptide 19 [Homo sapiens] (CYP2C19) (alternate designations include RP11-400G3.4; CPCJ; CYP 2C; CYP2C; P450C2C; P450IIC19; OTTHUMP00000059588; S-mephenytoin 4-hydroxylase; cytochrome P-450 II C; cytochrome P450, subfamily IIC (mephenytoin 4-hydroxylase), polypeptide 19; flavoprotein-linked monooxygenase; mephenytoin 4'-hydroxylase; microsomal monooxygenase; xenobiotic monooxygenase) maps to chromosome 10q24.1-10q24.3 (about nucleotides 96295564-96819172 of Build 36.1). Examples of nucleic acid sequences comprising CYP2C19 include those found in GenBank under accession numbers NM_000796, X65962, M61854, BC 111846. CYP2C 19 encodes a member of the cytochrome P450 superfamily of enzymes. CYP2C19 localizes to the endoplasmic reticulum and is involved in the metabolism of xenobiotics, including some barbituates.

ATP-binding cassette, sub-family B (MDR/TAP) member 4 [Homo sapiens] (ABCB4) (alternate names and abbreviations include ATP-binding cassette, subfamily B, member 4; P glycoprotein 3/multiple drug resistance 3; P-glycoprotein-3/multiple drug resistance-3; multidrug resistance protein 3; multiple drug resistance 3; ABC21; MDR2/3; MDR3; PFIC-3; PGY3) maps to chromosome 7q21.1 (about nucleotides 86869297-86947684 of Build 36.1). Examples of nucleic acid sequences comprising ABCB4 include those found in GenBank under accession numbers NM_01850, NM_018849, NM_000433, Z35284, M23234, BC020618. ABCB4 is a membrane-associated protein in the superfamily of ATP-binding cassette transporters. ABCB4 is part of the MDR/TAP subfamily, and may be involved in multidrug resistance as well as antigen presentation.

ATP-binding cassette, sub-family C (CFTR/MRP), member 3 [Homo sapiens] (ABCC3) (alternate names and abbreviations include ATP-binding cassette, sub-family C, member 3; canicular multispecific organic anion transporter; multidrug resistance associated protein; ABC31; MLP2; MOAT-D; MRP3; cMOAT2) maps to chromosome 17q22 (about nucleotides 45979561-46185071 of Build 36.1). Examples of nucleic acid sequences comprising ABCC3 include those found in GenBank under accession numbers NM_003786, Y17151, BC104952, BC050370, AF154001. ABCC3 is a member of the superfamily of ATP-binding cassette transporters. ABCC3 is a member of the MRP subfamily, and may be involved in multi-drug resistance.

Uridine diphosphate (UDP) glucuronosyltransferase 1 [Homo sapiens] (alternate names and abbreviations include UDP glucuronosyltransferase 1; UDP glucuronosyltransferase 1 family; UGT1A(x) where x is a number from 1-13) encodes several UDP-glucuronosyltransferases. The gene locus maps to chromosome 2q37 (about nucleotides 32902-188563 of Build 36.1). Examples of nucleic acid sequences comprising UGT1A(x) include those found in GenBank under accession numbers NM_000463, NM_019093, NM_007120, NM_019078, NM_205862, NM_001072, NM_019077, NM_019076, NM_021027, and NM_019075. The locus has 13 alternate first exons, 4 of which are considered pseudogenes, followed by 4 common exons. The 9 first exons may be spliced to the four common exons, giving rise to 9 transcripts encoding 9 different polypeptides having unique N-termini and common C-termini. The 9 transcripts include UGT1A1, UGT1A3, UGT1A4, UGT1A5, UGT1A6, UGT1A7, UGT1A8, UGT1A9 and UGT1A10. UGT1A1 and related transcript encode enzymes involved in the transformation of small lipophilic molecules (e.g. steroids, bilirubin, hormones, drugs and the like) into water-soluble excretable metabolites.

### METHODS

### PATIENT RECRUITMENT AND SAMPLE COLLECTION

The GATC ADR surveillance network consists of 10 full-time surveillors in major teaching hospitals across Canada serving approximately 75% of all Canadian children: IWK Grace Health Centre, Halifax NS; Montreal Children's Hospital, Montreal QC; Children's Hospital of Eastern Ontario, Ottawa ON; The Hospital for Sick Children, Toronto ON; Children's Hospital of Western Ontario, London Health Sciences Centre, London ON; Winnipeg Children's Hospital, Winnipeg MB and Alberta Children's Hospital, Calgary AB. A core group of 3 clinicians is located at Children's and Women's Health Centre of British Columbia (C&W).

The 10 clinical surveillors identified and recruited patients from inpatient wards, emergency departments, pediatric cancer wards, and ambulatory clinics. Biological samples (blood, saliva, buccal swabs) were collected from two groups of patients: (1) adverse drug reaction (ADR) patients, who experienced a serious or life-threatening adverse ADR that are identified by the GATC hospital-based pharmacists; (2) drug-matched control patients who receive the target drug but do not experience an ADR that are recruited by GATC clinical pharmacists. When feasible, samples are collected from parents of ADR patients at the same time as the ADR patients.

For each identified ADR case, the clinicians completed an electronic ADR report, provided patients/guardians with information about the study, and obtained patient/parent consent for sample and data collection (including Personal Health Number (PHN) for BC patients). Control patients were recruited by the clinicians using the same method as outlined for ADR patients, using the same demographic information (age, sex and ethnicity) and patient drug therapy information.

### CLINICAL SURVEILLANCE PERSONNEL TRAINING

The surveillance training included ADR identification, reporting, patient enrolment, ethical issues, obtaining informed consent, advertising the project within institutions, linkage with other healthcare professionals in the institutions and data transfer. Training was provided mostly via telephone and conference calls.

Each surveillor and site investigator was provided with the GATC Training and Reference Manual, a 65-page binder that outlines procedures and protocols: project advertisement, local laboratory setup for blood draws, DNA sample requirements and stability, DNA sample collection instructions (blood, buccal swab, and saliva cup), shipping instructions, instructions for data entry into the ADR database, target drug lists, and an extensive reference list on the subjects of ADRs, pharmacogenomics, and ADR surveillance. The ADR surveillance clinicians were also provided with template files for the documents created in the process of establishing Children's and Women's Health Centre of BC as the GATC index surveillance site.

### Ethical Approval

Ethical approval was obtained from the University of British Columbia's Clinical Research Ethics Board, the Children's and Women's Health Centre of BC ethics board as well as the local Institutional Review Board (IRB) for each clinical surveillance site.

### CLINICAL ADR DATABASE

The GATC Team developed a custom Filemaker (Santa Clara, CA USA)-based ADR database for collection of demographic and clinical information. This is a secure, password protected database that is accessible by all of the geographically-separated surveillance sites across Canada. The ADR database captures relevant clinical information about patients, ADRs, suspected drugs, concurrent medications, past and current medical conditions, ethnicity, as well as other relevant patient medical information.

### BIOLOGICAL SAMPLING

GATC surveillors collected 5 ml of whole blood, or 2 ml of saliva, or 2 buccal swabs from each ADR case and control. Each sample was identified with a unique GATC ID number. Blood was collected in a K2 EDTA tube following standard phlebotomy procedures at each site; samples were stored at 4°C. Saliva was collected using an Oragene™ kit (DNA Genotek), following manufacturer's protocol; samples were stored at room temperature. Buccal swabs were collected using the BuccalAmp™ kit (Epicentre Biotechnologies), following manufacturer's protocol; samples were stored at room temperature.

### DNA PURIFICATION

Blood samples were received and the bar-coded GATC ID labels on the tubes were scanned to input the new samples into the genomics database. DNA was purified and stored in tubes with unique laser-etched 10-digit bar-coded labels on the bottom of the tubes, which are linked to the GATC ID number in the database. DNA was purified from blood and buccal swabs using the Qiagen™ QiaAmp™ DNA purification kit and DNA was purified from saliva samples using the Oragene™ kit protocol.

### GENOTYPING

DNA samples were genotyped on the Illumina 500GX genotyping platform using the Illumina GoldenGate custom SNP genotyping assay to query the genotypes of 1536 single nucleotide polymorphisms (SNPs), following manufacturer's protocols (Illumina BeadStation 500G Genotyping System Manual, Illumina Document #11165222 Rev. A, 2004).

A secure database was created for storage of genotype data. This database is compatible with the raw Illumina data output.

### GATC ADME SNP PANEL

The GATC SNP panel was developed to represent the genetic variation in 220 key ADME genes, involved in drug absorption, distribution, metabolism, elimination, drug targets, drug receptors, transporters and the like. The genes include cytochrome P450 genes (CYP2D6, 2C9, 2C19, 3A4, 3A5, 1A1), N-Acetlytransferase (NAT1, NAT2), glutathione S-transferase (GSTM1, GSTM3, GSTT1, GSTP1), histamine methyltransferase (HMT), thiopurine methyltransferase (TPMT), ATP-binding cassette, sub-family B members (ABCB1 (MDR1), ABCC1, ABCC2 (MRP1, MRP2)) nuclear receptor subfamily 1, group I, member 2 (NR1I2; also called PXR or SXR) and many additional key drug metabolizing, target or receptor genes.

### IDENTIFICATION OF ADR-ASSOCIATED SNPs

Case-control association tests were used to test SNP association between ADR cases and controls. An estimate of the allelic odds ratio (OR) of developing the ADR in exposed (to the SNP) and unexposed patients were computed and the level of significance determined with a χ² test.

### ASSESSMENT OF CARDIOTOXICITY

Cardiac assessment for anthracycline compound-induced cardiotoxicity was performed at baseline and then before each cycle of treatment. At baseline, patients are assessed by ECG and echo and in some cases, MUGA. At cumulative anthracycline doses < 300 mg/m2, patients are assessed by echo before every second cycle of treatment. At higher cumulative doses (>300 mg/m2), patients are assessed by echo and MUGA before each cycle.

### EXAMPLE - INCIDENCE OF CARDIOTOXICITY IN ANTHRACYCLINE-TREATED SUBJECTS

Permanent and potentially life-threatening cardiotoxicity occurs in 6-10% of patients receiving standard doses of anthracyclines. Genetic variation in 220 drug metabolism genes was assessed in 16 patients that suffered permanent anthracycline-induced cardiotoxicity compared to 33 drug-matched controls. Twenty genetic variants were found to be highly predictive of susceptibility to anthracycline cardiotoxicity (TABLE 1). For example, patients with the "C" variant of the "T/C" SNP "rs 138054" on chromosome 22 at position 42544473 had a 5.3-fold higher odds of developing severe anthracycline-induced cardiotoxicity compared to patients that carry the "T" variant (P = 0.00015). The genomic DNA sequence surrounding these SNPs is shown in TABLE 2.

### ITEMIZED LISTING OF EMBODIMENTS

1. A method of screening a subject having a neoplastic disease for cardiotoxicity risk, the method comprising: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for the subject, where the subject is a candidate for anthracycline administration.
2. The method of embodiment 1, wherein the anthracycline is selected from one or more of the following: anthracycline antibiotics such as daunorubicin (daunomycin, rubidomycin), doxorubicin, idarubicin, epirubicin, mitoxantrone, carminomycin, esorubicin, quelamycin, aclarubicin, esorubicin, zorubicin, pirarubicin, amrubicin, iododoxorubicin, mitoxantrone and valrubicin.
3. The method of embodiment 1 or 2, wherein the method further comprises administering the anthracycline in accordance with the subject's risk of developing cardiotoxicity.
4. The method of any one of embodiments 1-3, wherein the identity of a single nucleotide polymorphism is determined by one or more of the following techniques:
   (a) restriction fragment length analysis;
   (b) sequencing;
   (c) micro-sequencing assay;
   (d) hybridization;
   (e) invader assay;
   (f) gene chip hybridization assays;
   (g) oligonucleotide ligation assay;
   (h) ligation rolling circle amplification;
   (i) 5' nuclease assay;
   (j) polymerase proofreading methods;
   (k) allele specific PCR;
   (l) matrix assisted laser desorption ionization time of flight (MALDI-TOF) mass spectroscopy;
   (m) ligase chain reaction assay;
   (n) enzyme-amplified electronic transduction;
   (o) single base pair extension assay; and
   (p) reading sequence data.
5. A method of determining cardiotoxicity risk from anthracycline administration, the method comprising: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for a subject receiving or about to receive one or more anthracyclines.
6. The method of embodiment 5, wherein the anthracycline is selected from one or more of the following: anthracycline antibiotics such as daunorubicin (daunomycin, rubidomycin), doxorubicin, idarubicin, epirubicin, mitoxantrone, carminomycin, esorubicin, quelamycin, aclarubicin, esorubicin, zorubicin, pirarubicin, amrubicin, iododoxorubicin, mitoxantrone and valrubicin.
7. The method of embodiment 5 or 6, wherein the method further comprises administering the anthracycline in accordance with the subject's risk of developing cardiotoxicity.
8. The method of any one of embodiments 5-7, wherein the identity of a single nucleotide polymorphism is determined by one or more of the following techniques:
   (a) restriction fragment length analysis;
   (b) sequencing;
   (c) micro-sequencing assay;
   (d) hybridization;
   (e) invader assay;
   (f) gene chip hybridization assays;
   (g) oligonucleotide ligation assay;
   (h) ligation rolling circle amplification;
   (i) 5' nuclease assay;
   (j) polymerase proofreading methods;
   (k) allele specific PCR;
   (l) matrix assisted laser desorption ionization time of flight (MALDI-TOF) mass spectroscopy;
   (m) ligase chain reaction assay;
   (n) enzyme-amplified electronic transduction;
   (o) single base pair extension assay; and
   (p) reading sequence data.
9. A method of treating a neoplastic disease in a subject in need thereof, the method comprising administering to the subject one or more anthracyclines, wherein said subject has a reduced risk of developing cardiotoxicity, wherein cardiotoxicity is based on the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto.
10. A method of treating a neoplastic disease in a subject in need thereof, the method comprising:
   (a) selecting a subject having a reduced risk of developing cardiotoxicity, wherein cardiotoxicity is based on the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto; and
   (b) administering to said subject one or more anthracyclines.
11. A method of selecting a chemotherapeutic regimen for a subject, the chemotherapeutic regimen comprising one or more anthracyclines, the method comprising: determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for the subject to assess the risk of cardiotoxicity.
12. A use of an anthracycline in the manufacture of a medicament for the treatment of neoplastic disease, wherein the subjects treated have a reduced cardiotoxicity risk genotype at one or more of the following polymorphic sites: rs138054; rs2071885; rs1229863;
   rs10509681; rs6499244; rs17863783; rs4148919; rs7785246; rs35607; rs16968478;
   rs11000122; rs4736349; rs741999; rs1677693; rs1845556; rs1149222; rs1910465;
   rs7441743; rs10786172; and rs2107441; or a polymorphic site in linkage disequilibrium thereto.
13. A use of an anthracycline in the manufacture of a medicament for the treatment of neoplastic disease in a subset of subjects, wherein the subset of subjects have a reduced cardiotoxicity risk genotype at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto.
14. A method of selecting test subjects to determine the efficacy of a therapeutic regimen known to be useful or suspected of being useful, for the treatment of a neoplastic condition, the method comprising:
   (a) determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for a test subject, where the test subject is a candidate for anthracycline administration; and
   (b) separating test subjects based on their risk of cardiotoxicity.
15. A method of determining risk of cardiotoxicity for a therapeutic regimen known to be useful or suspected of being useful, for the treatment of a neoplastic condition, the method comprising:
   (a) determining the identity of a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto, for a test subject, where the test subject is a candidate for anthracycline administration; and
   (b) separating test subjects based on their risk of cardiotoxicity prior to anthracycline administration.
16. A method for selecting a group of subjects for determining the side effects of a candidate drug known or suspected of being useful for the treatment of a neoplastic condition, the method comprising: determining a subject's genotype for a single nucleotide polymorphism (SNP) at one or more of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs 10786172; and rs2107441; or a polymorphic site in linkage disequilibrium thereto, for each subject, wherein a subject's genotype is indicative of the subject's risk of cardiotoxicity following chemotherapeutic regimen administration; and sorting subjects based on genotype.
17. The method of embodiment 16 further comprising, administering the candidate drug to the subjects or a subset of subjects and assessing the degree of hearing loss in each subject.
18. The method of embodiment 17, further comprising comparing the degree of hearing loss in response to the candidate drug based on genotype of the subject.
19. Two or more oligonucleotides or peptide nucleic acids of about 10 to about 400 nucleotides that hybridize specifically to a sequence contained in a human target sequence consisting of a subject's cardiotoxicity associated gene sequence, a complementary sequence of the target sequence or RNA equivalent of the target sequence and wherein the oligonucleotides or peptide nucleic acids are operable in determining the presence or absence of two or more polymorphism(s) in the cardiotoxicity associated gene sequence selected from of the following polymorphic sites: rs138054; rs2071885; rs1229863; rs10509681; rs6499244; rs17863783; rs4148919; rs7785246; rs35607; rs16968478; rs11000122; rs4736349; rs741999; rs1677693; rs1845556; rs1149222; rs1910465; rs7441743; rs10786172; and rs2107441; or a polymorphic site in linkage disequilibrium thereto.
20. Two or more oligonucleotides or peptide nucleic acids selected from the group consisting of:
   (a) an oligonucleotide or peptide nucleic acid that hybridizes under high stringency conditions to a nucleic acid molecule comprising SEQ ID NO:1 having an A at position 121 but not to a nucleic acid molecule comprising SEQ ID NO:1 having a G at position 121;
   (b) an oligonucleotide or peptide nucleic acid that hybridizes under high stringency conditions to a nucleic acid molecule comprising SEQ ID NO:1 having a G at position 121 but not to a nucleic acid molecule comprising SEQ ID NO:1 having an A at position 121;
   (c) an oligonucleotide or peptide nucleic acid that hybridizes under high stringency conditions to a nucleic acid molecule comprising SEQ ID NO:2 having a G at position 121 but not to a nucleic acid molecule comprising SEQ ID NO:2 having a C at position 121;
   (d) an oligonucleotide or peptide nucleic acid that hybridizes under high stringency conditions to a nucleic acid molecule comprising SEQ ID NO:2 having a C at position 121 but not to a nucleic acid molecule comprising SEQ ID NO:2 having a G at position 121;
   (e) an oligonucleotide or peptide nucleic acid that hybridizes under high stringency conditions to a nucleic acid molecule comprising SEQ ID NO:3 having an A at position 121 but not to a nucleic acid molecule comprising SEQ ID NO:3 having a T at position 121;
   (f) an oligonucleotide or peptide nucleic acid that hybridizes under high stringency conditions to a nucleic acid molecule comprising SEQ ID NO:3 having a T at position 121 but not to a nucleic acid molecule comprising SEQ ID NO:3 having an A at position 121;
   (g) an oligonucleotide or peptide nucleic acid that hybridizes under high stringency conditions to a nucleic acid molecule comprising SEQ ID NO:4 having a G at position 121 but not to a nucleic acid molecule comprising SEQ ID NO:4 having an A at position 121;
   (h) an oligonucleotide or peptide nucleic acid that hybridizes under high stringency conditions to a nucleic acid molecule comprising SEQ ID NO:4 having an A at position 121 but not to a nucleic acid molecule comprising SEQ ID NO:4 having a G at position 121;
   (i) an oligonucleotide or peptide nucleic acid that hybridizes under high stringency conditions to a nucleic acid molecule comprising SEQ ID NO:5 having an A at position 121 but not to a nucleic acid molecule comprising SEQ ID NO:5 having a T at position 121;
   (j) an oligonucleotide or peptide nucleic acid that hybridizes under high stringency conditions to a nucleic acid molecule comprising SEQ ID NO:5 having a T at position 121 but not to a nucleic acid molecule comprising SEQ ID NO:5 having an A at position 121;
   (k) an oligonucleotide or peptide nucleic acid that hybridizes under high stringency conditions to a nucleic acid molecule comprising SEQ ID NO:6 having an A at position 101 but not to a nucleic acid molecule comprising SEQ ID NO:6 having a C at position 101;
   (1) an oligonucleotide or peptide nucleic acid that hybridizes under high stringency conditions to a nucleic acid molecule comprising SEQ ID NO:6 having a C at position 101 but not to a nucleic acid molecule comprising SEQ ID NO:6 having an A at position 101;
   (m) an oligonucleotide or peptide nucleic acid that hybridizes under high stringency conditions to a nucleic acid molecule comprising SEQ ID NO:7 having an A at position 101 but not to a nucleic acid molecule comprising SEQ ID NO:7 having a G at position 101;
   (n) an oligonucleotide or peptide nucleic acid that hybridizes under high stringency conditions to a nucleic acid molecule comprising SEQ ID NO:7 having a G at position 101 but not to a nucleic acid molecule comprising SEQ ID NO:7 having an A at position 101;
   (o) an oligonucleotide or peptide nucleic acid that hybridizes under high stringency conditions to a nucleic acid molecule comprising SEQ ID NO:8 having a G at position 121 but not to a nucleic acid molecule comprising SEQ ID NO:8 having a C at position 121;
   (p) an oligonucleotide or peptide nucleic acid that hybridizes under high stringency conditions to a nucleic acid molecule comprising SEQ ID NO:8 having a C at position 121 but not to a nucleic acid molecule comprising SEQ ID NO:8 having a G at position 121;
   (q) an oligonucleotide or peptide nucleic acid that hybridizes under high stringency conditions to a nucleic acid molecule comprising SEQ ID NO:9 having an A at position 121 but not to a nucleic acid molecule comprising SEQ ID NO:9 having a G at position 121;
   (r) an oligonucleotide or peptide nucleic acid that hybridizes under high stringency conditions to a nucleic acid molecule comprising SEQ ID NO:9 having a G at position 121 but not to a nucleic acid molecule comprising SEQ ID NO:9 having an A at position 121;
   (s) an oligonucleotide or peptide nucleic acid that hybridizes under high stringency conditions to a nucleic acid molecule comprising SEQ ID NO:10 having an A at position 101 but not to a nucleic acid molecule comprising SEQ ID NO:10 having a G at position 101;
   (t) an oligonucleotide or peptide nucleic acid that hybridizes under high stringency conditions to a nucleic acid molecule comprising SEQ ID NO:10 having a G at position 101 but not to a nucleic acid molecule comprising SEQ ID NO:10 having an A at position 101; (u) an oligonucleotide or peptide nucleic acid that hybridizes under high stringency conditions to a nucleic acid molecule comprising SEQ ID NO:11 having an A at position 121 but not to a nucleic acid molecule comprising SEQ ID NO:11 having a G at position 121;
   (v) an oligonucleotide or peptide nucleic acid that hybridizes under high stringency conditions to a nucleic acid molecule comprising SEQ ID NO:11 having a G at position 121 but not to a nucleic acid molecule comprising SEQ ID NO:11 having an A at position 121;
   (w) an oligonucleotide or peptide nucleic acid that hybridizes under high stringency conditions to a nucleic acid molecule comprising SEQ ID NO:12 having an A at position 121 but not to a nucleic acid molecule comprising SEQ ID NO:12 having a G at position 121;
   (x) an oligonucleotide or peptide nucleic acid that hybridizes under high stringency conditions to a nucleic acid molecule comprising SEQ ID NO:12 having a G at position 121 but not to a nucleic acid molecule comprising SEQ ID NO:12 having an A at position 121;
   (y) an oligonucleotide or peptide nucleic acid that hybridizes under high stringency conditions to a nucleic acid molecule comprising SEQ ID NO:13 having an A at position 121 but not to a nucleic acid molecule comprising SEQ ID NO:13 having a G at position 121;
   (z) an oligonucleotide or peptide nucleic acid that hybridizes under high stringency conditions to a nucleic acid molecule comprising SEQ ID NO:13 having a G at position 121 but not to a nucleic acid molecule comprising SEQ ID NO:13 having an A at position 121;
   (aa) an oligonucleotide or peptide nucleic acid that hybridizes under high stringency conditions to a nucleic acid molecule comprising SEQ ID NO:14 having an A at position 121 but not to a nucleic acid molecule comprising SEQ ID NO:14 having a C at position 121;
   (bb) an oligonucleotide or peptide nucleic acid that hybridizes under high stringency conditions to a nucleic acid molecule comprising SEQ ID NO:14 having a C at position 121 but not to a nucleic acid molecule comprising SEQ ID NO:14 having an A at position 121;
   (cc) an oligonucleotide or peptide nucleic acid that hybridizes under high stringency conditions to a nucleic acid molecule comprising SEQ ID NO:15 having an A at position 121 but not to a nucleic acid molecule comprising SEQ ID NO:15 having a G at position 121;
   (dd) an oligonucleotide or peptide nucleic acid that hybridizes under high stringency conditions to a nucleic acid molecule comprising SEQ ID NO:15 having a G at position 121 but not to a nucleic acid molecule comprising SEQ ID NO:15 having an A at position 121; (ee) an oligonucleotide or peptide nucleic acid that hybridizes under high stringency conditions to a nucleic acid molecule comprising SEQ ID NO:16 having an A at position 121 but not to a nucleic acid molecule comprising SEQ ID NO:16 having a C at position 121;
   (ff) an oligonucleotide or peptide nucleic acid that hybridizes under high stringency conditions to a nucleic acid molecule comprising SEQ ID NO:16 having a C at position 121 but not to a nucleic acid molecule comprising SEQ ID NO:16 having an A at position 121;
   (gg) an oligonucleotide or peptide nucleic acid that hybridizes under high stringency conditions to a nucleic acid molecule comprising SEQ ID NO:17 having a G at position 121 but not to a nucleic acid molecule comprising SEQ ID NO:17 having an A at position 121;
   (hh) an oligonucleotide or peptide nucleic acid that hybridizes under high stringency conditions to a nucleic acid molecule comprising SEQ ID NO:17 having an A at position 121 but not to a nucleic acid molecule comprising SEQ ID NO:17 having a G at position 121;
   (ii) an oligonucleotide or peptide nucleic acid that hybridizes under high stringency conditions to a nucleic acid molecule comprising SEQ ID NO:18 having an A at position 101 but not to a nucleic acid molecule comprising SEQ ID NO:18 having a G at position 101;
   (jj) an oligonucleotide or peptide nucleic acid that hybridizes under high stringency conditions to a nucleic acid molecule comprising SEQ ID NO:18 having a G at position 101 but not to a nucleic acid molecule comprising SEQ ID NO:18 having an A at position 101;
   (kk) an oligonucleotide or peptide nucleic acid that hybridizes under high stringency conditions to a nucleic acid molecule comprising SEQ ID NO: 19 having an A at position 101 but not to a nucleic acid molecule comprising SEQ ID NO:19 having a G at position 101;
   (11) an oligonucleotide or peptide nucleic acid that hybridizes under high stringency conditions to a nucleic acid molecule comprising SEQ ID NO:19 having a G at position 101 but not to a nucleic acid molecule comprising SEQ ID NO:19 having an A at position 101;
   (mm) an oligonucleotide or peptide nucleic acid that hybridizes under high stringency conditions to a nucleic acid molecule comprising SEQ ID NO:20 having an A at position 121 but not to a nucleic acid molecule comprising SEQ ID NO:20 having a G at position 121; and
   (nn) an oligonucleotide or peptide nucleic acid that hybridizes under high stringency conditions to a nucleic acid molecule comprising SEQ ID NO:20 having a G at position 121 but not to a nucleic acid molecule comprising SEQ ID NO:20 having an A at position 121.
21. An array of oligonucleotides or peptide nucleic acids attached to a solid support, the array comprising two or more of the oligonucleotides or peptide nucleic acids of embodiment 19 or 20.
22. A composition comprising an addressable collection of two or more oligonucleotides or peptide nucleic acids, the two or more oligonucleotides or peptide nucleic acids consisting essentially of two or more nucleic acid molecules set out in SEQ ID NO:1-20 or compliments, fragments, variants, or analogs thereof.
23. The oligonucleotides or peptide nucleic acids of any one of embodiments 21 to 22, further comprising one or more of the following: a detectable label; a quencher; a mobility modifier; a contiguous non-target sequence situated 5' or 3' to the target sequence or 5' and 3' to the target sequence.

## Claims

1. A method of determining cardiotoxicity risk for a subject from anthracycline administration, the method comprising:
determining the identity of a single nucleotide polymorphism (SNP) at the polymorphic site rs17863783, for a subject receiving or about to receive one or more anthracyclines; and
determining whether the identity of the SNP is the risk genotype rs17863783T or the reduced risk genotype rs17863783G.

2. The method of claim 1, wherein said subject has a neoplastic disease.

3. The method of any one of claims 1-2, wherein the anthracycline is selected from one or more of the following: anthracycline antibiotics such as daunorubicin (daunomycin, rubidomycin), doxorubicin, idarubicin, epirubicin, mitoxantrone, carminomycin, esorubicin, quelamycin, aclarubicin, esorubicin, zorubicin, pirarubicin, amrubicin, iododoxorubicin, mitoxantrone and valrubicin.

4. The method of any one of claims 1-3, wherein the identity of a single nucleotide polymorphism is determined by one or more of the following techniques:
(a) restriction fragment length analysis;
(b) sequencing;
(c) micro-sequencing assay;
(d) hybridization;
(e) invader assay;
(f) gene chip hybridization assays;
(g) oligonucleotide ligation assay;
(h) ligation rolling circle amplification;
(i) 5' nuclease assay;
(j) polymerase proofreading methods;
(k) allele specific PCR;
(l) matrix assisted laser desorption ionization time of flight (MALDI-TOF) mass spectroscopy;
(m) ligase chain reaction assay;
(n) enzyme-amplified electronic transduction;
(o) single base pair extension assay; and
(p) reading sequence data.

5. One or more anthracyclines for use in a method of treating a neoplastic disease in a subject in need thereof, the method comprising:
(a) selecting a subject having a reduced risk of developing cardiotoxicity, wherein the reduced cardiotoxicity risk is based on the identity of a single nucleotide polymorphism (SNP) at the polymorphic site rs17863783 being the reduced risk genotype rs17863783G; and
(b) administering to said subject one or more anthracyclines.

6. One or more anthracyclines for use in a method of treating a neoplastic disease according to claim 5, wherein said method further comprises administering the anthracycline(s) in accordance with the subject's risk of developing cardiotoxicity.

7. A method of determining risk of cardiotoxicity for an anthracycline therapeutic regimen known to be useful or suspected of being useful for the treatment of a neoplastic condition, the method comprising:
(a) determining the identity of a single nucleotide polymorphism (SNP) at the polymorphic site rs17863783 for a test subject, where the test subject is a candidate for anthracycline administration;
(b) determining whether the identity of the SNP is the risk genotype rs17863783T or the reduced risk genotype rs17863783G; and
(c) separating test subjects based on their risk of cardiotoxicity prior to anthracycline administration.

8. A method for selecting a group of subjects for determining the side effects of an anthracycline therapeutic regimen comprising an anthracycline candidate drug known or suspected of being useful for the treatment of a neoplastic condition, the method comprising:
determining a subject's genotype for a single nucleotide polymorphism (SNP) at the polymorphic site rs17863783, for each subject, wherein a subject's genotype is indicative of the subject's risk of cardiotoxicity following anthracycline therapeutic regimen administration;
determining whether the identity of the SNP is the risk genotype rs17863783T or the reduced risk genotype rs17863783G; and
sorting subjects based on genotype.

9. The method of claim 8, further comprising assessing the degree of hearing loss in each subject following administration of the anthracycline candidate drug to the subject(s) or a subset of subjects, and optionally comparing the degree of hearing loss in response to the anthracycline candidate drug based on genotype of each subject.

10. Use of two or more oligonucleotides or peptide nucleic acids of about 10 to about 400 nucleotides that hybridize specifically to a sequence contained in a human target sequence consisting of a subject's cardiotoxicity associated gene sequence, a complementary sequence of the target sequence or RNA equivalent of the target sequence and wherein the oligonucleotides or peptide nucleic acids are operable in determining the presence or absence of two or more polymorphism(s) in the cardiotoxicity associated gene sequence of the polymorphic site rs17863783, for determining anthracycline-induced cardiotoxicity risk.

11. Use according to claim 10, wherein the two or more oligonucleotides or peptide nucleic acids are selected from the group consisting of:
(k) an oligonucleotide or peptide nucleic acid that hybridizes under high stringency conditions to a nucleic acid molecule comprising SEQ ID NO:6 having an A at position 101 but not to a nucleic acid molecule comprising SEQ ID NO:6 having a C at position 101; and
(I) an oligonucleotide or peptide nucleic acid that hybridizes under high stringency conditions to a nucleic acid molecule comprising SEQ ID NO:6 having a C at position 101 but not to a nucleic acid molecule comprising SEQ ID NO:6 having an A at position 101.

12. Use of an array of oligonucleotides or peptide nucleic acids attached to a solid support, the array comprising two or more of the oligonucleotides or peptide nucleic acids of claim 10 or 11, for determining anthracycline-induced cardiotoxicity risk.

13. Use of a composition comprising an addressable collection of two or more oligonucleotides or peptide nucleic acids, the two or more oligonucleotides or peptide nucleic acids consisting essentially of two or more nucleic acid molecules set out in SEQ ID NO:6 or complements thereof, for determining anthracycline-induced cardiotoxicity risk.

14. Use of the oligonucleotides or peptide nucleic acids of any one of claims 10-13, further comprising one or more of the following: a detectable label; a quencher; a mobility modifier; a contiguous non-target sequence situated 5' or 3' to the target sequence or 5' and 3' to the target sequence, for determining anthracycline-induced cardiotoxicity risk.


SEQUENCE LISTING

<110>  The University of British Columbia
       HAYDEN, Michael R.
       CARLETON, Bruce
       ROSS, Colin J.

<120>  POLYMORPHISMS PREDICTIVE OF ANTHRACYCLINE-INDUCED CARDIOTOXICITY

<130>  80021-991

<140>  PCT/CA2007/002065
<141>  2007-11-15

<150>  60/858,924
<151>  2006-11-15

<160>  20

<170>  PatentIn version 3.3

<210>  1
<211>  241
<212>  DNA
<213>  Homo sapiens

<400>  1
tgttccctat ccccttagag cacgcttggg cctgaactga tcttgggttc tgtcgcctgt      60

ctaccccact ggaatgcaaa ctccacagag gcagggcttt ctctgtcccc tgccgcatcc     120

rcagtgccgg gaacagtgcc tggcatggag caggtgcagt cgaatgaccg actccatgaa     180

gtgaggtgag tcacccacca cgcagggaag agtggacatg ggtaaacagg cgctgcctgg     240

g                                                                     241


<210>  2
<211>  241
<212>  DNA
<213>  Homo sapiens

<400>  2
cccagcaccc agctctcctg tctgtctccc agcagacctc ctccccatcc cagctcccac      60

ccactgctac tcaacctaaa cctgcttgtc tgtcagccac tgacactagc agccccacag     120

sctggccttg tctgtcccct acatcgacat ggtttccagc tggcactcag gcctctgccc     180

cacaggagca acctcacagt ccacatctgc accccatcgt ctctcctctt tcccatgggc     240

c                                                                     241


<210>  3
<211>  241
<212>  DNA

<213>  Homo sapiens

<400>  3
ctggatggtc ttcatctctt gaccttgtga tcctcctgcc tcggcctccc aaagtgctgg      60

gattacaggc atgcacctcc cattctgaac gggaggtaga ctttgcataa aaaactgcat     120

wtgaaaatcc tagagggcct tcaaactcat caagtccatt tcccaaactt taccattgtg     180

atggtttgaa tattagtcct tcaaatctca atttgaaatt tacttgccat tgtaatagta     240

t                                                                     241


<210>  4
<211>  241
<212>  DNA
<213>  Homo sapiens

<400>  4
ctacgtgatg tccactactt ctcctcactt ctggacttct ttataaatca gattatctgt      60

tttgttactt ccagggcaca accataatgg cattactgac ttccgtgcta catgatgaca     120

ragaatttcc taatccaaat atctttgacc ctggccactt tctagataag aatggcaact     180

ttaagaaaag tgactacttc atgcctttct cagcaggtaa tagaaactcg tttccatttg     240

t                                                                     241


<210>  5
<211>  241
<212>  DNA
<213>  Homo sapiens

<400>  5
aaagaaggtt taaatggaaa aagtaaaagc tgggacctac tgaaactatt tgtttatgtg      60

tttttggaaa tagtgaaaaa tgaaattata aatgataaaa aggcactttt ccatagcatt     120

wgatctagat ataaaatatt aatttaaaaa atttcctgaa agagaaagta aaaacataat     180

tttattaata aatgaggcta gaatggcata aaaataaatg gggattaaag aagtaattag     240

g                                                                     241


<210>  6
<211>  201
<212>  DNA
<213>  Homo sapiens


<220>
<221>  misc_feature
<222>  (187)..(187)
<223>  n= a or g or t or c

<400>  6
tatccacatc tctcttgagg acagctgatg cgagttcttc atactttgaa aacagacaat      60

aaaatagata gggctccaac aaattaacaa ggaagttggc mactcgttgg gaaaaagtca     120

tgtggtctga aaactttgtg tagcacctgg gaatgtagga cacagggtct gggcttctgc     180

tgaatgnatg ctccagggaa c                                               201


<210>  7
<211>  201
<212>  DNA
<213>  Homo sapiens

<400>  7
tcggtaaaaa gaaagcaagt gggtgacctg cgaggtcaca gtcagttctt catcctctgg      60

tgggtgacac tgcttcgtga ctggcaggtt ctagatttct rttctcgggg cctcttcaga     120

cacatcaaac ctgcaggaca ggccctcagc ttctctccgt gtctctaaca agcccctgga     180

agtctccaga ggaaagccag g                                               201


<210>  8
<211>  241
<212>  DNA
<213>  Homo sapiens

<400>  8
ataaggctac cagggttccc atagaccgcg tacttccctt cagtggcaag cgatagttgt      60

taggtttgtt gttattatgg cactagggtt aggggaagag ttcattcaat cattttattt     120

stgggagagt cacttccttc ccgggaaggc atcgctgagc acccactcac gcagctcagt     180

aatggacaca catcaatctg atgttttgct ttgtttatac aacaaataag attcttcttt     240

c                                                                     241


<210>  9
<211>  241
<212>  DNA
<213>  Homo sapiens

<400>  9
aaatggtggg aatacaggca tgagccgccg cgcccggctg taaggttggg gtttctcaaa      60

gcgtgggtgg tcaagaccta tcagtgggtt gtaaaatcaa tcaagtcaga caagagctac     120

rttttttaaa aaaaggatag gaatggcgga ggaacaggct gaagagaaca gagtatttcg     180

gaaagattct tccagagcat gggaaagccc tgctgcatta aacttgattc atatactcaa     240

g                                                                     241

<210>  10
<211>  201
<212>  DNA
<213>  Homo sapiens

<400>  10
cctgcctccc acattctgta actatcctat cattctcaga gtagagggtg tacacattta      60

gacccatcta aacgttgcct aggacgggga acatgatttt rttcattgca ggcagcttgg     120

atggataaac agaaaatcca aatcacagac ttaaagaaga tagaagtgta ttctctctcc     180

cataaaggat gtccaaggct g                                               201


<210>  11
<211>  241
<212>  DNA
<213>  Homo sapiens

<400>  11
gctcccaatg gcctgaaaat ggcacttttg atttcaatat acttagagac ttagacgact      60

tttgccatcg cagtgggaaa tggtctgaga ttccttatgt tcaggctttc ttcactctcc     120

rtaaccgccc ttccctctgc cagtactgct ccactttcca aatcctactc accagacttg     180

tctcccacca cccccgtctc ccaccacccc cgcaacagcc ccagccgaca actcctcttt     240

c                                                                     241


<210>  12
<211>  241
<212>  DNA
<213>  Homo sapiens

<400>  12
acatggtgct ggcatcttat cagcttctgg tgaggcctga ggaagtttac aatcatggag      60

gaaggcaaag ggggagccag cgtaccagat gacgagagtg ggcatgagaa agcgaggggg     120

raagtgccac attcttttga acaacagatc tcatgtgaac tcagataact tattctctac     180

taaggcgaca ctctcagcca ttcatgaggg atctgccccc atgatccagt acctcccacc     240

a                                                                     241


<210>  13
<211>  241
<212>  DNA
<213>  Homo sapiens

<400>  13
gctccgctcc ttccctcctc tgtaaaatgg ggacrggaga agtacttatg tctccgggtg      60

gtcatgcagt cccatctgta acaaacacag ccctaaaatc tccatgaggg caaggactct 120

rctcacccta catcccagtg cccagaacag agcccggcat acagtaggtg ctcagtactg 180

ctcaatacac ggccactacc gtcgtctgca attatagagt tttttgatgg gcagcatgag 240

a 241

<210> 14
<211> 241
<212> DNA
<213> Homo sapiens

<400> 14
tttaaaataa ttataggaaa gaaattcagc agtgaaatat gtgactatat tggatgcttg 60

ctatttgtca tttactgtat ttctagcgct tgtgccatgt aagggctgat gaactcaaac 120

mgtagctcag ctttttgcat gctctttttt acccacctag aggtccagtg tttcctgtga 180

tacttacctc taaactgcta aatagccaca ttttacggaa gcatattctt catacctttg 240

c 241

<210> 15
<211> 241
<212> DNA
<213> Homo sapiens

<400> 15
ttactcatta aaatgtaacg ttttctaaca cctgtaggat gtacatcgcc ctcaggagtt 60

cttaaacaaa aggatcaaat cacaggggca gaatttggtg atagctctta cctgacccca 120

rtatcatgat aataccaaaa ctttacctgc aactcttaat tgtatttatt cctgatcaca 180

atattaatgc caaattaatt accaatgaaa aacaccttct gtatgagttt atcatcttcc 240

a 241

<210> 16
<211> 241
<212> DNA
<213> Homo sapiens

<400> 16
ttatcttttt cactttcatg ttgtagccag actgagaatc ttatgatttc tatatgggat 60

ggggggtaga taaattatcg gtgggaccaa aattgacagt atattttgct gtggttcccc 120

mtctccaaaa aattatcact gcatgtttgg tatgatttta aataaactac attaaataaa 180

ctgactacag tgaaaaagaa aaagttttga cgtaaaaaag taagtcactg cattcaaata 240

t 241

<210> 17
<211> 241
<212> DNA
<213> Homo sapiens

<400> 17
tggcagtgag atcagaggtc tgatagagag caggacatag aaggtgatct gcctgatcag 60

gtaattagtt atatacaaaa ggggaattac ctggctgagg tagggtttct ccactccaaa 120

rttactcttc ccctcatact gcacttgccc tttataagaa agtcccaata tggatctcac 180

acttaaggaa tggcacgggg aactatacat aagtaggtga aattcttctg tatggtatat 240

t 241

<210> 18
<211> 201
<212> DNA
<213> Homo sapiens

<400> 18
acaagataat taaactaggt aattttctga aagaagttag aataatgtgg gcaaaaatgt 60

aggcaaagtg tttgtgcctt gaaaaaaaat acatatattc rtatataggc ataatttaat 120

ttttatgtat tatatatttt acccatgtgt atttacaaga aaggcaaagt ggtgagagaa 180

tcatgaggga aaactgcatc c 201

<210> 19
<211> 201
<212> DNA
<213> Homo sapiens

<400> 19
gcaggaagag acaacataag ttggcctggg attgcatgtt ggttttatta ttaaaaggcc 60

attttgaaca gcaagtgtat tgttaaagat aatttttctc rttctcaaaa cttcaggttc 120

aaatgctgga gtagggaaaa tggaaactct tcttattgaa ggataaatgg taatcctaga 180

aatgtagtga ggctgcctga a 201

<210> 20
<211> 241
<212> DNA
<213> Homo sapiens

<400> 20
gaaaacaaaa aggttcccct attccctccc cagcagccct ctctgtggcc tggagggttt 60

ctctggaaga gggcagtgcc tggaggggag cctctggata gtgatgtgga aatattaaca 120

rcagagtcaa agcgggtatt ttggggtagt cggggagaaa actgtgacct ctccccccaa 180

tgatgtaaaa tctttatcaa tgatggaaac tgttgtgtct cattaaaagg gttttgaggt 240

g 241